(19) Europäisches Patentamt
European Patent Office
Office européen des brevets

(11) Publication number : 0 500 297 A1

(12) EUROPEAN PATENT APPLICATION

(21) Application number : 92301283.5

(22) Date of filing : 17.02.92

(51) Int. Cl.[5] : C07D 239/36, C07D 213/64, C07D 213/69, C07D 213/80, C07D 213/82, C07D 215/22, C07D 401/10, C07D 401/12, C07D 403/10, C07D 405/06, C07D 405/14

(30) Priority : 16.02.91 GB 9103326
15.06.91 GB 9112975
21.06.91 GB 9113492
10.07.91 GB 9114829
28.09.91 GB 9120677
14.11.91 GB 9124168
26.11.91 GB 9125059
12.12.91 GB 9126575
12.12.91 GB 9126573
04.01.92 GB 9200101

(43) Date of publication of application : 26.08.92 Bulletin 92/35

(84) Designated Contracting States : PT

(71) Applicant : FISONS plc
Fison House Princes Street
Ipswich Suffolk IP1 1QH (GB)

(72) Inventor : Bantick, John Raymond
78 Melton Road, Burton on the Wolds
Loughborough, Leicestershire LE12 5HG (GB)
Inventor : McInally, Thomas
17 Belvoir Drive
Loughborough, Leicestershire LE11 2SN (GB)
Inventor : Tinker, Alan Charles
97 Knightthorpe Road
Loughborough, Leicestershire LE11 0JR (GB)
Inventor : Hirst, Simon Christopher
1 Blake Road
West Bridgford, Nottingham NG2 5JJ (GB)

(74) Representative : Gilholm, Stephen Philip
Fisons plc 12 Derby Road
Loughborough, Leicestershire LE11 0BB (GB)

(54) **Substituted pyridinones and pyrimidinones as angiotensin II antagonists.**

(57) There are described substituted heterocycles of general formula I,

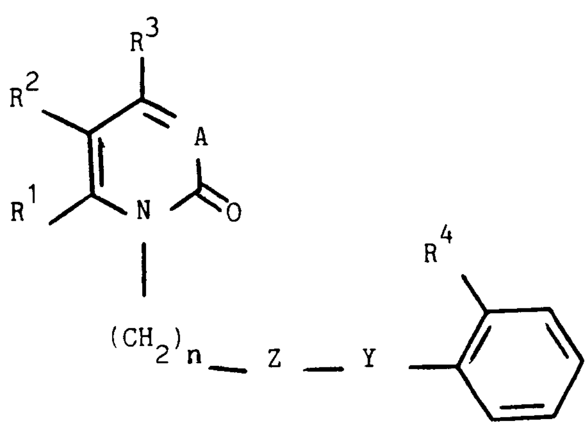

I

which are useful as angiotensin II antagonists
there are also described methods for making the compounds and pharmaceutical formulations, eg for the treatment of hypertension, comprising them.

EP 0 500 297 A1

Compounds

This invention relates to new compounds, methods for their preparation and compositions containing them.

1-(2′-Tetrazolylbiphenyl)pyrimidinones and 1-(2′-carboxybiphenyl)pyrimidinones are known from European Patent Application No: 0419048. Such compounds are useful as angiotensin II antagonists. Angiotensin II is an arterial vasoconstrictor which interacts with cell membrane receptors. Angiotensin II receptor antagonism is thought therefore to be useful in the prevention or alleviation of, inter alia, hypertension.

Several other compounds have been reported to be useful as angiotensin II antagonists, eg in EP 399731 and the imidazole benzofuran derivatives of EP 434249, the imidazole benzothiophen derivatives of EP 430709 and the imidazole indole derivatives of EP 429257. More recently, pyrimidine and triazoline phenylacetic acid derivatives have been described in WO 91/12001 as being useful as angiotensin II antagonists.

We have now found a group of novel compounds which have advantageous properties eg as angiotensin II receptor blockers.

According to the invention we provide compounds of formula I,

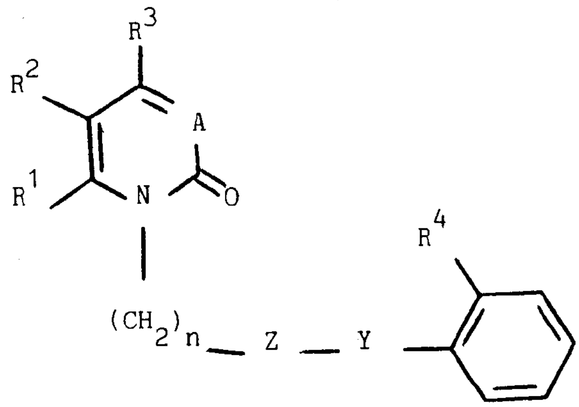

I

in which A is -N- or $-CR^5-$,

$R^2$ is hydrogen, alkyl C1 to 6, halogen or $-COOR^{21}$, or together $R^1$ and $R^2$ form a chain $-B=CR^7-CR^8=CR^9-$, in which B is in the 8- position,

B is -N- or $-CR^6-$,

$R^6$, $R^7$, $R^8$ and $R^9$, which may be the same or different, are each hydrogen, alkyl C1 to 6, alkoxy C1 to 6, $-S(O)_qR^{22}$ or $-COOR^{23}$,

$R^3$ is hydrogen, hydroxy, alkyl C1 to 6, alkoxy C1 to 6, $-(CH_2)_rCOOR^{10}$ $-(CH_2)_tOR^{31}$ or $-NR^{26}R^{27}$

$R^5$ is hydrogen, alkyl C1 to 6, alkanoyl C2 to 7, phenyl, halogen, nitrile, nitro $-NR^{24}R^{25}$ $-CONR^{11}R^{12}$, $-(CH_2)_mOR^{13}$ or $-COOR^{14}$,

Z is a group of formulae II or III

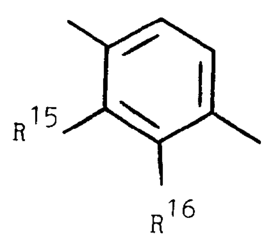

or

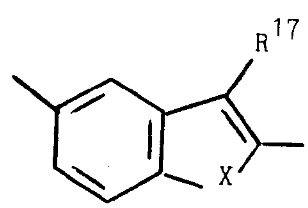

II

III

X is -O-, -S- or $-NR^{18}-$,
$R^{15}$ and $R^{16}$ are each hydrogen or together form a chain -CH=CH-CH=CH-,
$R^{17}$ is hydrogen, halogen, alkyl C1 to 6 or nitrile,
Y is $-(CH_2)_s-$, $-OCHR^{20}-$, $-SCHR^{20}-$, or

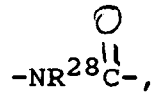

one of $R^4$ and $R^{20}$ is -COOH or tetrazolyl and the remainder is hydrogen.

$R^{12}$, $R^{24}$ and $R^{27}$, which may be the same or different, are each hydrogen, alkyl C1 to 6, alkanoyl C2 to 7, phenyl, $-(CH_2)_pCOOR^{30}$ or phenylalkyl C7 to 12,

$R^{10}$ $R^{14}$ and $R^{30}$ which may be the same or different, are each hydrogen, alkyl C1 to 6, phenyl, phenylalkyl C7 to 12 or $-(CH_2)_lCH(phenyl)_2$,

$R^{11}$, $R^{13}$, $R^{18}$, $R^{21}$, $R^{23}$, $R^{25}$, $R^{28}$, $R^{31}$ and $R^{32}$, which may be the same or different, are each hydrogen or alkyl C1 to 6, or $R^{11}$ and $R^{12}$ may together form a group $-CH_2CH_2MCH_2CH_2-$

$R^{26}$ is hydrogen, alkyl C1 to 6 or $-COR^{29}$

$R^1$, $R^{22}$ and $R^{29}$, which may be the same or different, are each alkyl C1 to 6,

M is -O- or $-NR^{32}-$,

n, m and p, which may be the same or different, are each an integer from 1 to 6, provided that when $R^{27}$ is $-(CH_2)_pCOOR^{30}$, p may be O,

l, r, s and t, which may be the same or different, are each an integer from 0 to 6, and,

q is O, 1 or 2,

and pharmaceutically acceptable salts, esters, amides, tautomers and enantiomers thereof.

According to the invention we also provide a process for the production of a compound of formula I or a pharmaceutically acceptable salt, ester, amide, enantiomer or tautomers thereof, which comprises

a) removal of a protecting group from a compound of formula I in which one or more of the amino, hydroxy, carboxylic acid or tetrazolyl groups is protected,

b) preparation of a compound of formula I which comprises reacting a compound of formula IV,

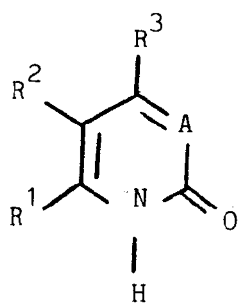

IV

in which A, $R^1$, $R^2$ and $R^3$ are as defined above,

with a compound of formula V

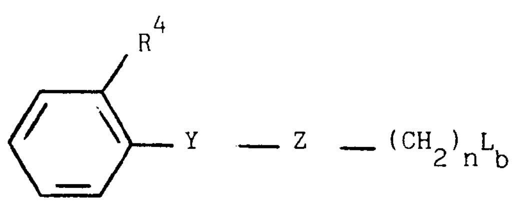

V

in which Y, Z, $R^4$ and n are as defined above, and $L_b$ is a leaving group

c) preparation of a compound of formula I in which

A is $-CR^5-$,

$R^1$ is alkyl C1 to 6,
$R^2$ is hydrogen, alkyl C1 to 6 or $-COOR^{21}$,
$R^3$ is hydroxy, and
$R^5$ is hydrogen, alkyl C1 to 6, phenyl or $-(CH_2)_mOR^{13}$, which comprises reacting a compound of formula VI,

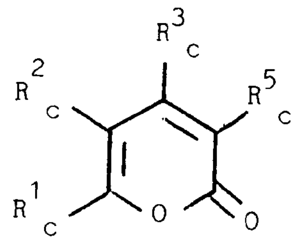

VI

in which $R^1_c$ is alkyl C1 to 6,
$R^2_c$ is hydrogen, alkyl C1 to 6 or $-COOR^{21}$
$R^3_c$ is hydroxy, and
$R^5_c$ is hydrogen, alkyl C1 to 6, phenyl or $-(CH_2)_mOR^{13}$,
with a compound of formula VII,

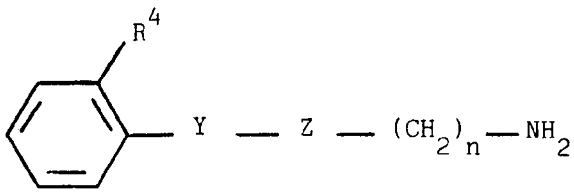

VII

in which Y, Z, $R^4$ and n are as defined above,
d) preparation of a compound of formula I in which A is $-CR^5-$,
$R^3$ is hydroxy,
$R^1$ and $R^2$ together form a chain $-CR^6=CR^7-CR^8=CR^9$, and
$R^5$ is hydrogen, alkyl C1 to 6, phenyl, $-NR^{24}R^{25}$ or $-(CH2)_mOR^{13}$,
which comprises, heating a compound of formula XV,

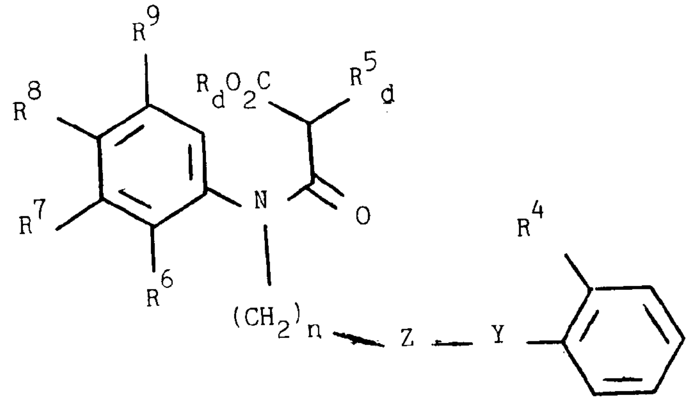

XV

in which Z, Y, n, $R^4$, $R^5$, $R^6$, $R^7$, $R^8$ and $R^9$ are as defined above,
$R^5_d$ is hydrogen, alkyl C1 to 6, phenyl $-NR^{24}R^{25}$ or $-(CH_2)_mOR^{13}$, and
$R_d$ is alkyl C1 to 6,
e) preparation of a compound of formula I in which at least one of $R^3$ or $R^5$ is amino, hydroxy or hydroxyalkyl

C1 to 6, which comprises reducing a compound of formula I in which at least one of $R^3$ and $R^5$ contains a group $-NO_2$, -COOH, $-OR_e$ or alkyl-$OR_e$,

wherein $R_e$ is alkyl C1 to 6,

f) preparation of a compound of formula I in which $R^7$ is $-S(O)_qR^{22}$- and q is 1 or 2, by oxidising a compound of formula I in which q is o,

g) preparation of a compound of formula I in which at least one of $R^2$ and $R^5$ represents halogen, which comprises halogenating a compound of formula I in which at least one of $R^2$ and $R^5$ is hydrogen,

h) preparation of a compound of formula I in which $R^5$ is $-COCH_3$, which comprises hydrolysing a compound of formula XII

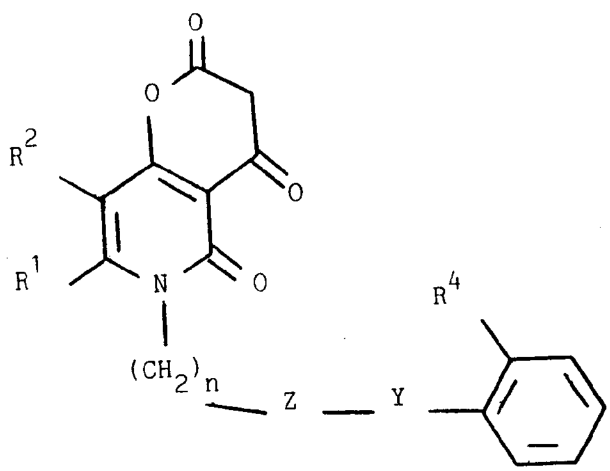

XII

in which Z, Y, $R^1$, $R^2$, $R^4$ and n are as defined above

i) preparation of a compound of formula I in which $R^4$ or $R^{20}$ is tetrazolyl, which comprises reacting a compound of VIII or IX,

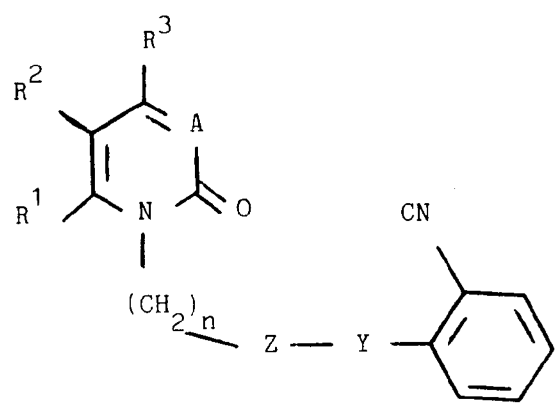

VIII

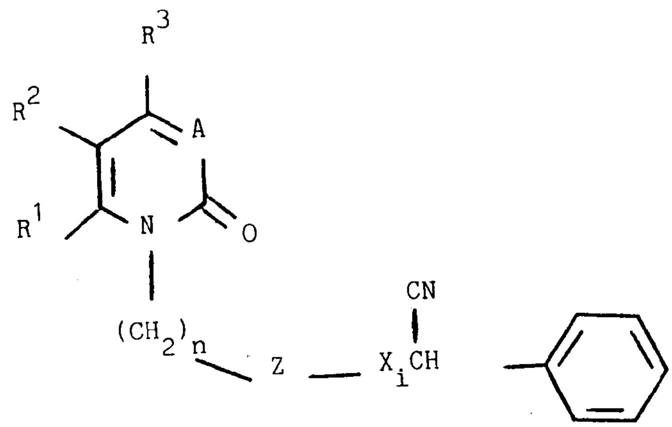

IX

in which A, Z, Y, $R^1$, $R^2$, $R^3$ and n are as defined above, and
$X_i$ is -O- or -S-,
with an azide.

j) preparation of a compound of formula I, in which one of $R^4$ and $R^{20}$ is -COOH, which comprises hydrolysing a compound of formula VIII or IX,

k) preparation of a compound of formula I in which $R^5$ is $-NO_2$ which comprises nitrating a compound of formulae I, VIII or IX, in which $R^5$ is hydrogen, and where necessary hydrolysing or forming a tetrazole of the resulting compound,

l) preparation of a compound of formula I in which one or more hydroxy, amino or carboxylic acid groups is alkylated or acylated which comprises reacting an appropriate compound of formula I in which the hydroxy, amino or carboxylic acid group is unalkylated or unacylated with an alkylating or and acylating group,

m) production of a pharmaceutically acceptable salt, ester, amide, enantiomer or tautomer of a compound of formula I, by treating a compound of formula I, or another salt, an ester or an amide thereof, with a compound containing an available pharmaceutically acceptable ion and capable of converting the compound of formula I or the other salt, ester, amide, enantiomer or tautomer thereof, to a pharmaceutically acceptable salt of the compound of Formula I.

In process a) the protecting group can be any convenient protecting group conventionally used and may be removed using conventional techniques. Thus acid protecting groups which may be used are alkyl C1 to 6, which may be a straight chain or branched alkyl, eg t-butyl; or phenylalkyl C7 to 12, eg benzyl. These groups can be removed by hydrolysis, for example basic hydrolysis, eg using aqueous methanolic sodium hydroxide; or cleavage using, for example, trifluoracetic acid; or by hydrogenation, eg using palladium on charcoal. Other acid protecting groups include trialkylsilylalkyl groups, eg trimethylsilyl ethyl, which may be removed by fluoride ion decomposition.

Amino-protecting groups which may be mentioned include alkyloxycarbonyl C2 to 7, eg t-butyloxycarbonyl or phenylalkyloxycarbonyl C8 to 13, eg benzyloxycarbonyl. We prefer to use starting materials in which the carboxy groups are protected.

When one of $R^4$ or $R^{20}$ is tetrazolyl a suitable protecting group, such as trityl, benzhydryl, trialkyltin (for example trimethyltin or tributyltin) or triphenyltin, affixed to a nitrogen of the tetrazolyl may be used.

Deprotection of the tetrazolyl may be carried out by decomposition, for example, when the protecting group is trityl, benzhydryl, trialkyltin or triphenyltin, the decompostion conditions may include, for example, acid catalysed hydrolysis in a protic acid such as aqueous hydrochloric acid conventionally in an aqueous solvent such as aqueous dioxan or 2-propanol. Alternatively, a trityl or benzhydryl group may be removed by hydrogenolysis.

Hydroxy protecting groups include, for example, alkyl C1 to 6, especially methyl; phenylalkyl C7 to 12, especially benzyl; alkanoyl C2 to 6, such as acetyl and haloalkanoyl C2 to 6, especially trifluoroacetyl.

Removal of the hydroxy protecting group depends on the nature of the protecting group; conventional techniques may generally be employed, including acidic or basic cleavage or hydrogenolysis. For example, protecting alkyl or phenylalkyl groups may be removed by cleavage using a protic acid, eg hydrochloric acid or a hydrobromic acid at a temperature of from about 0 to 150°C, or a Lewis acid, eg by reacting with boron trihalide in a halocarbon solvent. 1-Phenylalkyl groups, eg, benzyl, may be removed by catalytic hydrogenation using a suitable catalyst, eg palladium, in a suitable solvent, eg methanol or acetic acid.

The reaction of process b) is preferably carried out in a polar solvent eg, N,N-dimethyl formamide at between 0° and the boiling point of the solvent. The reaction is preferably carried out in the presence of a base

eg a hydride, such as sodium hydride.

The reaction of process c) may be carried out in a polar solvent eg an alcohol such as ethanol, dimethyl formamide, or water or a mixture of polar solvents. The reaction may be carried out at an elevated temperature, eg the boiling point of the solvent.

The reaction of process d) may be carried out at elevated temperature eg in excess of 150° and preferably in excess of 200° eg up to 250°. Although a solvent may be used in the reaction depending upon the temperature required, it is preferred that the reaction is carried out in the absence of a solvent.

The reductions of process e) may be carried out using general reducing procedures known per se. The reducing agent may be, for example when reducing a carbonyl group, electrophiliceg diborane, or nucleophilic, eg a complex metal hydride such as sodium borohydride. Aprotic solvents are preferred, eg tetrahydrofuran, diethyl ether or 1,2-dimethoxyethane. The reaction is preferably carried out low temperature eg 0° to -30° eg -15°. For the reduction of eg nitro groups to amines, the reducing agent may be a metal in an acidic solvent, eg iron in glacial acetic acid, although other known reducing agents may also be used. Such reductions are preferably carried out at elevated temperature eg at the boiling point of the solvent.

The oxidation of process f) may be carried out using conventional oxidising agents, eg potassium peroxymonosulphate. The reaction is preferably carried out in a protic solvent, eg water, and at elevated temperature, eg the boiling point of the solvent.

The halogenation of process g) may be carried out using conventional halogenating agents. For example, dihalogenation may be achieved by reaction with the molecular halogen. Such reactions are preferably carried out at room temperature and in a protic solvent in which the halogen is soluble eg water. Monohalogenation can be achieved by reaction with a selective halogenating agent eg an N-haloamide or N-haloimide eg a succinimide. The preferred halogen in this case is bromine.

The reaction of process h) is preferably carried out under basic conditions eg using a metal hydroxide such as sodium hydroxide. The reaction may be carried out in a protic solvent or a mixture of protic solvents eg methanol and water. The reaction may be carried out at elevated temperature eg at the boiling point of the solvent.

The reaction of process i) may be carried out in an inert solvent eg toluene at elevated temperature. Any conventional azide may be used eg an azido alkyl tin such as azido trimethyl tin.

The rection of process j) may be carried out under basic conditions eg using a metal hydroxide such as sodium hydroxide. The reaction is preferably carried out in a protic solvent eg water, or a mixture of solvents, and preferably at elevated temperature, eg the boiling point of the solvent.

The nitration of process k) is preferably carried out under acidic conditions using nitric acid and an acidic solvent eg an alkanoic acid such as acetic acid or a protic acid such as sulphuric acid. The reaction is preferably carried out at elevated temperature, eg the boiling point of the solvent.

In process l) conventional alkylating or acylating groups may be used eg such as those described under process a). Conventional alkylating conditions may be used eg alkylation may be under basic or acidic conditions and acylation may be in the presence of a catalyst eg a metal halide such as aluminium chloride.

In process m) the salts may be formed by reacting the free acid, or a salt, ester, amide or derivative thereof, or the free base, or a salt, ester, amide, enantiomer, tautomer or protected derivative thereof, with one or more equivalents of the appropriate base or acid. The reaction may be carried out in a solvent or medium in which the salt is insoluble or in a solvent in which the salt is soluble, eg water, dioxan, ethanol, tetrahydrofuran or diethyl ether, or a mixture of solvents, which may be removed in vacuo or by freeze drying. The reaction may also be a metathetical process or it may be carried out on an ion exchange resin.

Compounds of formulae IV, V and VII are either known or may be prepared from known compounds using conventional processes known per se. In particular, compounds of formula IV may be prepared from the methods disclosed in US Patent No: 4451469 and/or J. Med. Chem, (1985) **28**, page 1790.

Compounds of formulae V and VII wherein Z is a group of formula III may be prepared by the methods described in European patent Application Nos: 434249 (benzofurans), 429257 (indoles) and 430709 (benzothiophenes).

Compounds of formulae V and VII wherein Z is a group of formula II and Y is other than $-(CH_2)_s-$ may be prepared by the methods described in WO91/12001.

Compounds of formulae V and VII wherein Z is a group of formula II and Y represents $-(CH_2)_s-$ may be prepared by methods described in European Patent Application No. 0419048.

Compounds of formula VI are prepared by the methods described in J. Chem. Soc. (C), 1997 (1969), starting from acetone dicarboxylic acid, with the exception that in the initial step to produce the intermediate 3,5-diacyl-4,6-dihydroxy-2-pyrone a catalyst, eg conc. sulphuric acid, 1% mol/mol, and four equivalents of acid anhydride were used.

According to the invention we also provide the compounds of formulae VIII and IX which are useful as intermediates. We prefer the compounds of formulae VIII and especially those in which Z is a group II and Y is a bond.

The compounds of formulae VIII and IX may be prepared by analogous methods to those described for the preparation of compounds of formula I.

Compounds of formula XII may be prepared by the following reaction sequence, for example;

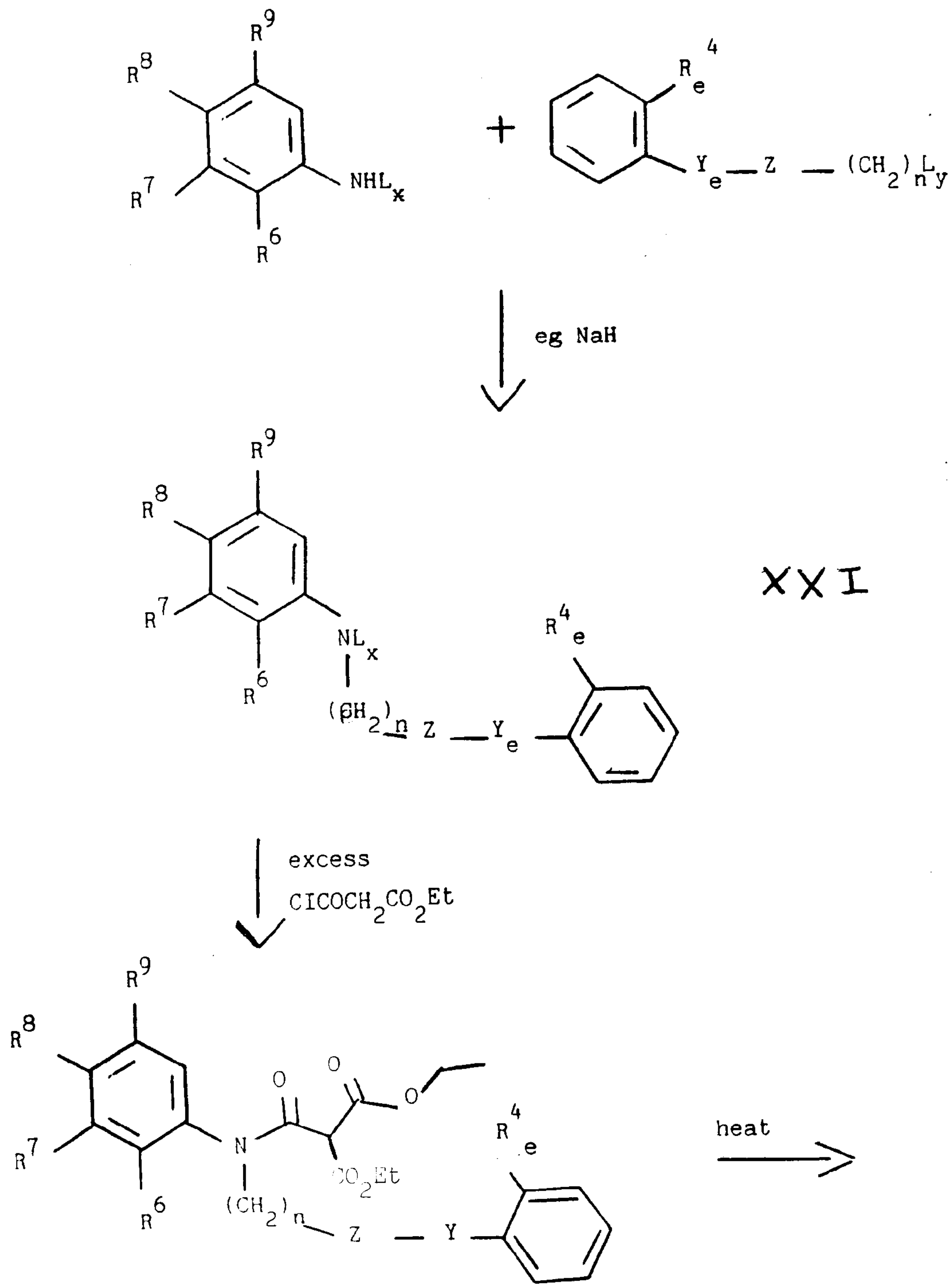


XXI

wherein Z, n, $R^6$, $R^7$, $R^8$ and $R^9$ are as defined above,

$R^4_e$ and $Y_e$ have the same significance as $R^4$ and Y save that any carboxylic acid or tetrazole groups are protected,

$L_y$ is a leaving group, eg halogen, and

$L_x$ is a protecting group eg $-COCF_3$, or hydrogen.

Compounds of formula XII may also be prepared using reagents similar to $ClCOCH_2CO_2Et$, such as

$halCOCH_2CO_2R_d$

in which $R_d$ is as defined above.

Similarly, compounds of formula XV may be prepared by reacting a compound of formula XXI with a compound of formula XXII

$halCOCHR^5_dCO_2R_d$ XXII

wherein $R^5_d$ and $R_d$ are as defined above, and

hal is halogen.

Pharmaceutically acceptable salts of the compounds of formula I include ammonium salts, alkali metal salts, eg sodium and potassium salts; alkaline earth metal salts, eg the calcium and magnesium salts; salts with organic bases, eg salts with dicyclohexylamine or N-methyl-D-glucamine; and salts with amino acids, eg with arginine, lysine etc. Also when the molecule contains a basic group, salts with organic or inorganic acids, eg with HC1, HBr, $H_2SO_4$, $H_3PO_4$, methanesulphonic, toluenesulphonic, maleic, fumaric or camphorsulphonic acids. The non-toxic physiologically acceptable salts are preferred, although other salts are also useful, eg in isolating or purifying the product.

Pharmaceutically acceptable esters include esters with C1 to 10 alcohols, eg alkyl C1 to 6 esters and esters with benzyl alcohol. The amides may be, for example, unsubstituted or mono-or di-C 1 to 6 alkyl amides and may be made by conventional techniques, eg reaction of an ester of the corresponding acid with ammonia or an appropriate amine.

According to a further feature of the invention we provide compounds of formula VI which are useful as intermediates.

Compounds of formula VI may be prepared by reacting a compound if formula XIII,

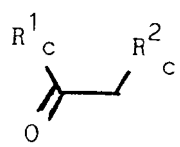

XIII

with a compound of formula XIV,

$L_cCOCHR^5_cCO_2H$ XIV

in which $R^1_c$, $R^2_c$ and $R^5_c$ are as defined above, and

$L_c$ is a leaving group.

Compounds of formulae XIII and XIV are either known per se or may be prepared from known intermediates using known processes.

A preferred group of compounds is compounds of formula I in which A is $-CR^5-$, and

B is $-CR^6-$.

A number of other groups of compounds of formula I may be mentioned as preferred groups. For example, a preferred group of compounds is compounds of formula I, or a salt, ester, amide, enantiomer or tautomer thereof,

in which A is $-CR^5-$,

n is 1,

Z is a group of formula II,

Y is a bond, and

$R^1$, $R^2$, $R^3$, $R^4$, $R^5$, $R^{15}$ and $R^{16}$ are as defined above.

The following subgroups of compounds are also preferred.

A compound of formula I, or a salt, ester, amide, enantiomer or tautomer thereof, in which

A is $-CR^5-$,

$R^1$ is alkyl C1 to 6,

$R^2$ is hydrogen, alkyl C 1 to 6, halogen or $-COOR^{21}$, and

Z, Y, $R^3$, $R^4$, $R^5$ and n are as defined above.

A compound of formula I, or a salt, ester, amide, enantiomer or tautomer thereof, wherein

A is $-CR^5-$,

$R^1$ and $R^2$ form a group $-CR^6=CR^7-CR^8=CR^9-$,

$R^7$ is hydrogen, alkyl C1 to 6, alkoxy C1 to 6, $S(O)_qR_{22}$ or $-COOR^{23}$,

$R^6$, $R^8$ and $R^9$ are each hydrogen, and

Z, Y, $R^3$, $R^4$ and $R^5$ are as defined above.

Compounds of formula I, or a salt, ester, amide, enantiomer or tautomer thereof, wherein

A is $-CR^5-$,
$R^1$ is alkyl C1 to 6,
$R^2$ and $R^5$ are each hydrogen,
$R^3$ is hydroxy, alkoxy C1 to 6 or $-(CH_2)_rCOOR^{10}$,
n is 1,
Z is a group of formula II,
Y is a bond, and
$R^4$, $R^{15}$ and $R^{16}$ are as defined above.

A further sub-group of compounds is compounds of formula I, or a salt, ester, amide, enantiomer or tautomer thereof, wherein Z is a group of formula III

A represents $-CR^5-$
n is 1,
Y is a bond, and
$R^1$, $R^2$, $R^3$ and $R^4$ are as defined above.

A further sub-group of compounds is compounds of formula I, or a salt, ester, amide, enantiomer, or tautomer thereof, wherein Z is a group of formula II,

A represents $-CR^5-$,
n is 1,
Y is $-OCHR^{20}-$, $-SCHR^{10}-$ or

$$-NR^{28}\overset{\overset{O}{\|}}{C}-,$$

and

$R^1$, $R^2$ and $R^3$ are as defined above, and
$R^4$ is hydrogen.

A further preferred group of compounds is compounds of formula I, or a salt, ester, amide, enantiomer or tautomer thereof wherein,

$R^1$ is propyl or ethyl,
$R^2$ is hydrogen or halogen, or together $R^1$ and $R^2$ form a group $-CR^6=CR^7-CR^8=CR^9-$,
$R^7$ is methoxy or ethoxy,
$R^6$, $R^8$ and $R^9$ are each hydrogen,
$R^3$ is hydrogen, hydroxy or $-(CH_2)_rCOOR^{10}$,
n is 1,
Z is a group of formula II,
Y is a bond, and
$R^4$ and $R^5$ are as defined above.

The following preferences may also be mentioned;

We prefer $R^1$ to be alkyl C1 to 6, when $R^1$ is alkyl C1 to 6 it is preferably C1 to 4, preferably butyl and especially propyl.

$R^2$ is preferably $-COOR^{21}$, halogen or alkyl C1 to 6, but especially hydrogen.

$R^5$ is preferably hydrogen.

n is preferably 1 to 2 and especially 1.

$R^{15}$ and $R^{16}$ are preferably both hydrogen.

$R^4$ is preferably tetrazolyl and especially tetrazol-5-yl.

$R^3$ is preferably hydrogen, alkyl C1 to 6, or $-NR^{26}R^{27}$, more preferably alkoxy C1 to 6 $-(CH_2)_rCOOR^{10}$, and especially hydroxy.

$R^7$ is preferably alkyl C1 to 6, eg ethyl, and especially alkoxy C1 to 6, eg methoxy or ethoxy,

$R^6$, $R^8$ and $R^9$ are preferably hydrogen.

$R^{21}$ is preferably alkyl C1 to 6, eg methyl or ethyl q, r, s and I are each preferably O.

$R^{22}$ is preferably alkyl C1 to 6, eg methyl.

$R^{10}$, $R^{26}$, $R^{27}$, $R^{24}$, $R^{25}$, $R^{13}$, $R^{15}$, $R^{16}$, $R^{18}$ and $R^{28}$ are each preferably hydrogen.

$R^{27}$ is preferably phenylalkyl C7 to 12, eg phenylmethyl or phenylethyl.

$R^{17}$ is preferably halogen and especially bromine

X is preferably -O-.

Y is preferably $-OCHR^{20}-$, $R^{20}$ is tetrazolyl, but Y is more preferably a bond ie $-(CH_2)_s-$ when s=O

$R^{29}$ is preferably ethyl and especially methyl.

$R^{30}$ is preferably phenylalkyl C7 to 12 eg phenylmethyl or phenylethyl.

By the term alkyl we mean straight, branched or cycloalkyl, eg containing up to and including 6 carbon atoms, but we especially prefer alkyl to mean straight chain alkyl.

Specific compounds which may be mentioned include,

4-hydroxy-6-propyl-1-({2′-[1H-tetrazol-5-yl][1,1′-biphenyl]-4-yl}methyl)-2(1H)-pyridinone,

6-butyl-1,2-dihydro-2-oxo-1-{(2′-[1H-tetrazol-5-yl][1,1′-biphenyl]-4-yl)methyl}pyridine-4-carboxylic acid,

6-butyl-4-hydroxy-1-{[2′-(1H-tetrazol-5-yl)[1,1′-biphenyl]-4-yl]methyl}-2(1H)-pyridinone.

The compounds of the invention are advantageous in that they are more efficaceous, produce less side effects, are longer acting, more readily absorbed, less toxic, distributed in the body tissues in a different manner or have other advantageous properties when compared to compounds of similar structure.

The compounds of the invention are useful because they possess pharmacological properties. In particular they antagonise the actions of angiotensin II (see Example A). Angiotensin II is a potent vasoconstrictor in mammals. It also stimulates aldosterone release which results in salt and fluid retention. Increased blood pressure is the physiological result of these changes. Antagonists of angiotensin II are thus effective antihypertensive agents in a variety of animal models (see Example D) and are indicated for use clinically in all conditions where antagonism of angiotensin II may be beneficial, for example, in patients with renovascular, malignant or essential hypertension or chronic congestive heart failure. See, for example, D W Cushman et al., Biochemistry 16, 5484 (1977) and E W Petrillo and M A Ondetti, Med. Res. Rev. _ 93 (1982).

More recently, angiotensin II antagonists have been found to be useful in treating disorders of the central nervous system.

Thus, the compounds of this invention are useful as antihypertensives in treating hypertensive mammals, including humans and they can be utilised to achieve reduction of blood pressure, eg in formulations containing appropriate pharmaceutically acceptable excipients, diluents or carriers. In particular, the compounds of the invention may be used in the treatment or prophylaxis of hypertension. The compounds of the invention may also be used as cognition enhancers, as anxiolytics and in the treatment or prophylaxis of cognitive disorders such as dementia (eg Alzheimer's disease), schizophrenia and other diseases such as renal failure, hyperaldosteronism, cardiac insufficiency, congestive heart failure, post-myocardial infarction, cerebrovascular disorders, perioperative organ protection, glaucoma, diabetic retinopathy and disorders of intracellular hemeostasis.

According to a further feature of the invention we provide the use of a compound of formula I or a pharmaceutically acceptable salt, ester, amide, enantiomer or tautomer thereof as a pharmaceutical, especially in the treatment of the aforementioned diseases, eg hypertension.

According to another feature of the invention we provide the use of a compound of formula I or a pharmaceutically acceptable salt, ester, amide, enantiomer or tautomer thereof in the manufacture of a medicament for the treatment of the aforementioned diseases and especially hypertension.

The compounds of the invention can be administered (to animals or humans) in unit dosages of 1 to 500mg generally given several times, eg 1 to 4 times, per day thus giving a total daily dose of from 1 to 2000 mg per day. The dose will vary depending on the type and severity of disease, weight of patient and other factors which a person skilled in the art will recognise.

The compounds of this invention may be given in combination with other pharmaceutically active compounds, eg diuretics or antihypertensives. The dosage of the other pharmaceutically active compound can be that conventionally used when the compound is administered on its own, but is preferably somewhat lower. To illustrate these combinations, one of the compounds of this invention effective clinically in the range, eg 1-200 milligrams per day, can be combined at levels ranging, eg from 1-200 milligrams per day with the following antihypertensives and diuretics in dose ranges per day as indicated:

hydrochlorothiazide (15-200mg), chlorothiazide (125-2000mg), ethacrynic acid (15-200mg), amiloride (5-20mg), furosemide (5-80mg), propanolol (20-480mg), timolol (5-50mg) nifedipine (20-100mg), verapamil (120-480mg) and methyldopa (65-2000mg). The above dose ranges may be adjusted on a unit basis as necessary to permit divided daily dosage. Also, the dose may vary depending on the severity of the disease, weight of patient and other factors which a person skilled in the art will recognise.

The compounds of the invention may also be combined with ACE inhibitors such as captopril, delapril hydrochloride, enalapril, enalaprilat, fosinopril sodium, lisinopril, pentopril, quinapril hydrochloride, ramapril and utibapril.

According to our invention we also provide a pharmaceutical formulation comprising a therapeutically effective amount of a compound of formula I, or a pharmaceutically acceptable salt, ester, amide, enantiomer or tautomer thereof, in admixture with a pharmaceutically acceptable adjuvant, diluent or carrier.

The formulation preferably comprises less than 80%, more preferably less than 50%, eg 1 to 20%, by weight of a compound of formula I.

Thus the compound may be put up as a tablet, capsule, dragee, suppository, suspension, solution, injection, implant, a topical, eg transdermal, preparation such as a gel, cream, ointment, aerosol or a polymer system, or an inhalation form, eg an aerosol or a powder formulation.

We prefer compositions which are designed to be taken oesophageally and to release their contents in the gastrointestinal tract. Thus we prefer tablets which may, for example, be made by direct compression. In such a process the active ingredient is mixed with one or more of modified forms of starch, calcium phosphate, a sugar eg lactose, microcrystalline cellulose and/or other directly compressible excipients, together with lubricant(s), eg stearic acid or magnesium stearate, flow aid(s), eg talc or colloidal silicon dioxide, and disintegrant(s), eg starch or the materials sold under the Trade Marks, Nymcel, Ac-Di-Sol, Explotab and Plasdone XL. Tablets are then formed by direct compression, and may be sugar or film coated eg with hydroxypropylmethylcellulose.

Alternatively the active ingredient may be granulated before tabletting. In such cases the active ingredient is mixed with one or more of starch, calcium phosphate, a sugar eg lactose, microcrystalline cellulose or other suitable excipients and granulated with a binder such as starch, pregelled starch, polyvinylpyrrolidone, gelatine, a modified gelatine, or a cellulose derivative, eg hydroxypropylmethylcellulose. The mass is then dried, sieved and mixed with lubricant(s), flow aid(s) and disintegrant(s), such as described in the previous paragraph.

Tablets are then formed by compression of the granules, and may be sugar or film coated, eg with hydroxypropylmethylcellulose.

As a further alternative a powder, blend or granules, such as are described above as intermedlates in tabletting, may be filled into a suitable, eg gelatine, capsule.

In order to improve the bioavailability, or decrease variability of availability, of the active ingredient the compound may be:-

a) dissolved in a suitable solvent, eg polyethylene glycol, Gelucaire, arachis oil, a (hydrogenated) vegetable oil or beeswax and the solution is then filled into a gelatine capsule,

b) produced as a spray-dried or freeze-dried form prior to mixing with other excipients,

c) milled and/or micronised to produce a powder with a large surface area prior to mixing with other excipients,

d) made into a solution and distributed over an inert excipient having a large surface area, eg colloidal silicon dioxide. The solvent is evaporated and further excipients added,

e) formed into a complex with cyclodextrin prior to mixing with other excipients. This complex also assists in increasing light stability, or

f) made into a solid solution or co-precipitated, eg with polyvinylpyrrolidone, polyethyleneglycol, modified cellulose, hydroxypropylmethylcellulose, urea or a sugar prior to mixing with further excipients.

The compounds, either in their normal form or in a modified form, eg as described immediately above, may be formulated in a controlled release form. Thus the compound may be dispersed, or contained in, a polymer matrix formed from, for example, ethylcellulose, hydroxypropylmethylcellulose or the product sold under the Trade Mark Eudragit. Alternatively the compound may be formulated as a tablet or beads which are surrounded by a semi-permeable membrane, eg shellac, ethylcellulose or an acrylate/methacrylate polymer.

Compounds of formulae I, IV, VIII and IX may exist in various tautomeric forms. For example compounds of formulae I, IV, VIII and IX in which $R^3$ is hydroxy may exist as the 2-hydroxy-4-one derivative. Similarly, compounds of formula IV may exist as the unsaturated 2-hydroxy derivative, eg

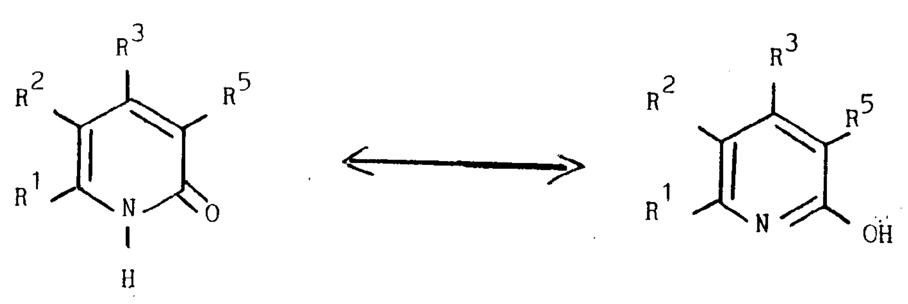

Compounds of formulae I, V, VII, XV, XII, VIII and IX may exist as different stereoisomers, in particular, compounds in which Y is $-OCHR^{20}$ or $-SCHR^{20}-$ may exist as different stereoisomers.

The invention will now be illustrated by the following Examples in which temperatures are in degrees Celcius.

Example 1

4′[(6-Butyl-3-cyano-1,2-dihydro-2-oxo-pyridin-1-yl) methyl]-[1,1′-biphenyl]-2-carboxylic acid dicyclohexylamine salt

(a) Methyl 4′-[(6-butyl-3-cyano-1,2-dihydro-2-oxo-pyridin-1-yl)methyl]-1,1′-biphenyl]-2-carboxylate

Sodium hydride (0.28g of 80% dispersion, 0.0009M), freed from oil, was suspended in dry dimethylformamide (20ml) under nitrogen.

6-Butyl-3-cyano-2(1H)-pyridinone (1.62g, 0.009M) in dry dimethylformamide (10ml) was added dropwise and the solution was stirred at room temperature for 30 minutes. Methyl 4′-bromomethyl-[1,1′-biphenyl]-2-carboxylate (3.09g; 0.01M) in dry dimethylformamide (10ml) was then added dropwise and the resulting solution was stirred at room temperature overnight.

The solution was treated with brine and extracted with ethyl acetate, which was then washed with brine and dried. Evaporation and chromatography (silica, 25% ethyl acetate/hexane) of the residue gave the subtitle compound (1.18g) as an oil.

(b) 4′-[(6-Butyl-3-cyano-1,2-dihydro-2-oxo-pyridin-1-yl) methyl]-[1,1′-biphenyl]-2-carboxylic acid dicyclohexylamine salt

The product from Example 1 (a) (1.1g; 0.0003M) was refluxed in 4/1 tetrahydrofuran/water (20ml) with lithium hydroxide monohydrate (0.12g; 0.003M) overnight.

The mixture was poured into water and extracted with ethyl acetate. The aqueous phase was acidified with 2N hydrochloric acid solution and extracted with ethyl acetate. The organic phase was then washed with brine. Evaporation and chromatography (silica, 3% ethanol/dichloromethane) of the residue gave the title acid (0.4g) as a yellow oil. The oil was then dissolved in ethanol (20ml) and dicyclohexylamine (one equivalent) was added. Evaporation and trituration with hexane yielded a yellow solid which was recrystallized from acetonitrile to give the title salt (0.49g) as yellow.crystals, mp 196-198°.

Anal found: C,76.1; H,7.7; N,7.6%

Calculated for $C_{36}H_{45}N_3O_3$: C,76.2; H,8.0; N,7.4%

Examples 2 to 3 were prepared by the method of Example 1.

Example 2

4′-[(3-Cyano-1,2-dihydro-6-methyl-2-oxo-pyridin-1-yl) methyl][1,1′-biphenyl]-2-carboxylic acid

mp 231-234°

Example 3

4′-[3-Cyano-1,2-dihydro-6-methyl-2-oxo-5-propylpyridin-1-yl]methyl[1,1′-biphenyl]-2-carboxylic acid

mp 186-187.5°

Example 4

6-Butyl-1-[(2′-carboxy[1,1′-biphenyl]-4-yl)methyl]-1,2-dihydro-2-oxopyridine-3-carboxylic acid

(a) Methyl 6-butyl-1,2-dihydro-2-oxopyridine-3-carboxylate

6-Butyl-3-cyano-2(1H)-pyridinone (9.0g) was refluxed in concentrated hydrochloric acid solution (100ml) for 6 hours. The solution was poured into water and extracted with ethyl acetate, which was then extracted with saturated sodium hydrogen carbonate solution. The aqueous phase was acidified with 2N hydrochloric acid solution and extracted with ethyl acetate, which was then washed with brine, dried and evaporated. Trituration of the residue with diethyl ether gave 6-butyl-1,2-dihydro-2-oxopyridine-3-carboxylic acid (7.03g) as a cream solid.

The acid (2g) methanol (100ml) and concentrated sulphuric acid solution (1ml) were refluxed overnight. The mixture was poured into water and basified with 2N sodium hydroxide solution, then extracted with ethyl

acetate. The organic phase was washed with brine. Evaporation and trituration in diethyl ether yielded the subtitle product (1.6g) as a cream solid.

(b) Methyl 6-butyl-1-[(2′-methoxycarbonyl[1,1′biphenyl]-4-yl)methyl]-1,2-dihydro-2-oxopyridine-3-carboxylate

The product from step (a) was reacted with methyl 4′-bromomethyl [1,1′-biphenyl]-2-carboxylate using the method of Example 1 (a) to give the subtitle compound (0.51g) as a colourless solid, mp 126-127°.

(c) 6-Butyl-1-[(2′-carboxy[(1,1′biphenyl]-4-yl)methyl]-1,2-dihydro-2-oxopyridine-3-carboxylic acid

The product from step (b) was hydrolysed using the method of Example 1 (b) to yield the title compound, recrystallized from ethyl acetate, (0.36g).
mp 185-187°
Anal found: C,70.8; H,5.8; N,3.1%
Calculated for $C_{24}H_{23}NO_5$: C,71.1; H,5.7; N,3.5%

Example 5

4′[(6-Butyl-1,2-dihydro-2-oxo-pyridin-1-yl)methyl] (1,1′biphenyl]-2-carboxylic acid

(a) 6-Butyl-1,2-dihydro-2-(1H)-pyridinone

6-Butyl-1,2-dihydro-2-oxopyridine-3-carboxylic acid (3.95g) was heated at 300° for 15 minutes. Chromatography (silica, 3% ethanol/dichloromethane) gave the subtitle compound (2.77g).
mp 70-71°

(b) Methyl 4′-[(6-butyl-1,2-dihydro-2-oxo-pyridin-1-yl)methyl][1,1′-biphenyl]-2-carboxylate

The product from step (a) was reacted with methyl 4′-bromomethyl[1,1′-biphenyl]-2-carboxylate using the method of Example 1 (a) to give the subtitle compound (1.5g) as a solid.

(c) 4′-[(6-Butyl-1,2-dihydro-2-oxo-pyridin-1-yl)methyl] [1,1′biphenyl]-2-carboxylic acid

The product from step (b) was hydrolysed using the method of Example 1 (b) to yield the title acid (recrystallized from ethyl acetate) (0.74g).
mp 169-171°
Anal found: C,76.5; H,6.4; N,3.9%
Calculated for $C_{23}H_{23}NO_3$; C,76.4; H,6.4; N,3.9.

Example 6 was made by the methods of Examples 5 and 8e)

Example 6

2-{4-[(6-Butyl-1,2-dihydro-2-oxo-pyridin-1-yl) methyl]naphthalene-1-yl}benzoic acid, sodium salt

m.pt 288° (dec)

Example 7

4′[(6-Butyl-1,2-dihydro-4-hydroxy-2-oxopyridine-1-yl) methyl][1,1′-biphenyl]-2-carboxylic acid

(a) Methyl 4′-[(6-butyl-1,2-dihydro-4-hydroxy-2-oxopyridin -1-yl)methyl][1,1′-biphenyl]-2-carboxylate

Water (10 ml) was added to a solution of methyl 4′-aminomethyl-[1,1′-biphenyl]-2-carboxylate (0.55 g) (prepared according to the method of EP 399731) and 6-butyl-4-hydroxy-2H-1-pyran-2-one (0.25 g) in dimethyl formamide (7 ml).

The solution was heated at reflux for 20 hrs. On cooling the solution was partitioned between ethyl acetate and 2N hydrochloric acid, followed by washing the organic phase with water and brine. Evaporation and chromatography (silica, 5% methanol/dichloromethane) gave the sub-title compound (0.41 g), mp 155.5-156°.

(b) 4′[(6-Butyl-1,2-dihydro-4-hydroxy-2-oxopyridin-1-yl) methyl] [1,1′-biphenyl]-2-carboxylic acid

The ester from step (a) was hydrolysed by the method of Example 1(b) to yield the title acid, mp 259-260°.

Example 8

Benzyl 6-butyl-1-[(2′-carboxy[1,1′-biphenyl]-4-yl)methyl]-1,2-dihydro-2-oxopyridine-3-carboxylate, sodium salt

a) Benzyl-6-butyl-1,2-dihydro-2-oxopyridine-3-carboxylate

To 6-butyl-1,2-dihydro-2-oxopyridine-3-carboxylic acid (1g) and triethylamine (0.72ml) in dry tetrahydrofuran (25ml) at -15° under nitrogen was added isobutyl chloroformate with stirring. After 2 minutes benzyl alcohol (0.53ml) was added. The mixture was stirred for 16 hours and then evaporated. The residue was partitioned between ethyl acetate and aqueous sodium hydrogen carbonate solution.

The organic phase was washed with dilute hydrochloric acid and brine, dried and evaporated to an oil, which was chromatographed (silica: dichloromethane/ethyl acetate (2%)) to give the sub-title compound as a solid (0.58g)

b) 2-Trimethylsilylethyl 4′-bromomethyl[1,1′-biphenyl]-2-carboxylate

4′-Methyl[1,1′-biphenyl]-2-carboxylic acid (12g), thionyl chloride (8.3ml) and dimethylformamide (2 drops) were refluxed in dichloromethane (200ml) for 3 hours.

The solvent was evaporated and the residue dissolved in dry pyridine (30ml). Then trimethylsilylethanol (8.9ml) in pyridine (30ml) was added dropwise with stirring. After 16 hours the mixture was added to dilute sulphuric acid and then extracted with ethyl acetate, which was washed with sodium hydrogen carbonate solution and brine. Drying and evaporation gave an oil which was chromatographed (silica: hexane/ethyl acetate (3%)) to afford 2-trimethylsilylethyl 4-methyl[1,1′biphenyl]-2-carboxylate as an oil (14.7). This oil, N-bromosuccinimide (8.39g), and azido bis(isobutyronitrile) (100mg) in dry dichloromethane (500ml) were refluxed for 16 hours under nitrogen whilst being illuminated by a halogen lamp. The resulting solution was washed with brine, and evaporated. The residue was chromatographed (silica: hexane/ethyl acetate (1%)) to give the sub-title ester as a waxy solid (14.9g).

MS: m/z 390/392 ($M^+$)

$^1$HNMR (salient peaks): δ 4.6 (s,2H,$CH_2Br$); 4.14 (m, 2H,$OCH_2$); 0.75 (m, 2H, $CH_2Si$); 0.0 (s, 9H,$Si(CH_3)_3$)

c) Benzyl 6-butyl-1,2-dihydro-2-oxo-1-{(2′-[2-trimethylsilylethoxycarbonyl][1,1′-biphenyl]-4-yl)methyl} pyridine-3-carboxylate

Benzyl 6-butyl-1,2-dihydro-2-oxo-pyridine-3-carboxylate (1.7g) was reacted with 2-trimethylsilyethyl 4′-bromomethyl[1,1′-biphenyl]-2-carboxylate (2.8g) by the method of Example 1 a) to give the sub-title product (1-37g),
mp 77-79°

d) Benzyl 6-butyl-1-[(2′carboxy[1,1′-biphenyl]-4-yl)methyl]-1,2-dihydro-2-oxopyridine-3-carboxylate

To the di-ester product from step c) (0.9g) in dry acetonitrile (20ml) was added tetrabutylammonium fluoride (3ml of 1M solution). The mixture was stirred for 16 hours, diluted with water and extracted with ethyl acetate. The ethyl acetate was washed with dilute hydrochloric acid and extracted with ethyl acetate. The ethyl acetate was washed with dilute hydrochloric acid, and brine, and evaporated to an oil, which was chromatographed (silica: dichloromethane/ethanol/formic acid 98/2/0.02) to give the sub-title product as a foam (0.49g)

e) Benzyl 6-butyl-1-[(2′-carboxy[1,1′biphenyl]-4-yl)methyl]1,2-dihydro-2-oxopyridine-3-carboxylate, sodium salt

The acid-ester product from step d) (0.49g) was dissolved together with sodium hydrogen carbonate (0.083g) in water (50ml) and dioxan (10ml). The resulting solution was freeze-dried to afford the title sodium salt as a solid.

MS: m/z 518 ($M^+$+1)

Anal Found C,65.5; H,5.2; N,2.6; Na, 4.4%

Calculated for $C_{31}H_{28}NO_5Na$:

C,65.16; H,5.95; N,2.45; Na,4.0%

Example 9 was prepared by the methods of Example 8 and Example 1b).

Example 9

Methyl 6-butyl-1-[(2′-carboxy[1,1′-biphenyl]-4-yl)methyl]-1,2-dihydro-2-oxopyridine-3-carboxylate dicyclohexylamine salt

mp 188-189°

Example 10

4′[(6-Butyl-1,2-dihydro-3-((phenylmethoxy) carbonylmethyl)aminocarbonyl-2-oxo-pyridin-1-yl)methyl][1,1′-biphenyl]-2-carboxylic acid

a) 6-Butyl-1,2-dihydro-2-oxo-1-[(2′-[2-trimethylsilylethoxy]carbonyl[1,1′-biphenyl]-4-yl)methyl] pyridine-3-carboxylic acid

Benzyl 6-butyl-1,2-dihydro-2-oxo-1-{(2′-[2-trimethylsilylethoxy]carbonyl[1,1′-biphenyl]-4-yl)methoxy} pyridine-3-carboxylate (0.2g) in ethanol (30ml) was hydrogenated over 10% palladium on carbon cataylst for 2 hours. The mixture was filtered, and the filtrate was evaporated to afford the sub-title acid-ester, (0.16g), mp 89-90°

b) 2-Trimethylsilylethyl 4′-[(6-butyl-1,2-dihydro-3-(phenylmethoxy)carbonylmethyl)aminocarbonyl-2-oxopyridin-1-yl]methyl[1,1′-biphenyl]-2-carboxylate

By the method of Example 8 a) the product from step a) was reacted with the benzyl ester of glycine to afford the sub-title product as an oil.

MS: m/z 653 ($M^+$1), 283 (BP)

$^1$HNMR (salient peaks): δ 8.5 (d,pyridone-H); 6.35(d, pyridone-H); 5.5 (bs, $NCH_2Ar$); 5.2 (s,$OCH_2Ph$); 4.3 (dd,$NHCH_2CO$); 4.15 (m, $CO_2CH_2$); 0.8 (m, $CH_2Si$)

c) 4′-[(6-Butyl-1,2-dihydro-3-((phenylmethoxy), carbonylmethyl)aminocarbonyl-2-oxopyridin-1-yl)methyl[1,1′-biphenyl]-2-carboxylic acid

The product from step b) was converted by the method of Example 8 d) to the title product, mp 159-160°

Anal Found C,72.0; H,6.0; N,5.3%

Calculated for $C_{33}H_{32}N_2O_6$

C,71.7; H,5.8; N,5.1%

mp 159-160°

Examples 11 to 15 were prepared by the method of Example 10

Example 11

4′-[(6-Butyl-1,2-dihydro-3-phenylmethylaminocarbonyl-2-oxo-pyridin-1-yl)methyl][1.1′-biphenyl]-2-carboxylic acid

mp 98-100°

Example 12

4′-[(6-Butyl-1,2-dihydro-3-(N-methoxycarbonylmethyl-N-methylamino)carbonyl-2-oxo-pyridin-1-yl)methyl][1,1′-biphenyl]-2-carboxylic acid

$C_{28}H_{30}N_2O_6$ requires:

C,68.55; H,6.2; N,5.7%

found: C,68.3; H 6.5; N5.6%

Example 13

4′-[(6-Butyl-1,2-dihydro-3-(4-morpholinyl)carbonyl-2-oxo-Pyridin-1-yl)methyl][1,1′-biphenyl]-2-carboxylic acid

m.pt 165-166°

Example 14

4′-[(6-Butyl-1,2-dihydro-3-(4-methyl-1-piperazinyl) carbonyl-2-oxo-pyridin-1-yl)methyl][1,1′-biphenyl]-2-carboxylic acid

mp 173-175°

Example 15

Ethyl 6-butyl-1-[2′-carboxy-[1,1′-biphenyl]-4-ylmethyl]-1,2-dihydro-2-oxo-pyridine-4-carboxylate

mp 80-85°

Example 16

4′-[(6-Butyl-1,2-dihydro-3-(diphenylmethoxy)carbonyl-2-oxo-pyridin-1-yl)methyl][1,1′-biphenyl]-2-carboxylic acid

6-Butyl-1,2-dihydro-2-oxopyridine-3-carboxylic acid (5.0g) and diphenyldiazomethane (28g) were refluxed under nitrogen in ethyl acetate for 3 hours. The solvent was evaporated, and the residue was chromatographed (silica: hexane/ethyl acetate (20%)) to give diphenylmethyl 6-butyl-1,2-dihydro-2-oxopyridine-3-carboxylate (7.6g).

mp 136-137°

This ester was converted in an analogous manner to Example 8 to the 2-trimethylsilyethyl ester of the title product,
mp 97-99°,

and then to the title acid-ester, which was obtained as a foam of indeterminate mp.

Anal: Found C,77.15; H,6.1; N,2.5%
Calculated for $C_{37}H_{33}NO_5$
C,77.7; H, 5.8; N,2.45%

Example 17

4′-[(6-Butyl-3-(carboxymethyl)aminocarbonyl-1,2-dihydro-2-oxo-pyridin-1-yl)methyl][1,1′-biphenyl]-2-carboxylic acid

4′-[(6-Butyl-1,2-dihydro-3-phenylmethoxycarbonylmethyl) aminocarbonyl-2-oxopyridin-1-yl)methyl[1,1-biphenyl]-2-carboxylic acid (0.2g) in ethanol (30ml) was hydrogenated over 10% palladium on charcoal for 4 hours.

Filtration and evaporation of the filtrate gave a foam, which was dissolved in sodium hydrogen carbonate

solution and washed with ethyl acetate. Acidification gave the title product.
mp 130° (dec)

Anal: Found: C,66.0; H,5.9; N5.58%

Calculated for $C_{26}H_{26}N_2O_6$. O.7EtOAc

C,66.0; H,6.1: N,5.4%

Examples 18 and 19 were prepared by the methods of Examples 10 and 17.

Example 18

4′-[(6-Butyl-3-(2-carboxyethyl)aminocarbonyl-1,2-dihydro-2-oxo-pyridin-1-yl)methyl][1,1′-biphenyl]-2-carboxylic acid

mp 186-187°

Example 19

4′-[(6-Butyl-3-(N-carboxymethyl-N-methyl) aminocarbonyl-1,2-dihydro-2-oxo-pyridin-1-yl)methyl][1,1′-biphenyl]-2-carboxylic acid

| | |
|---|---|
| | $C_{27}H_{28}N_2O_6$ $H_2O$ requires: |
| | C 65.6; H 6.1; N 5.7% |
| found: | C,65.6 H,6.3; N5.5% |

Example 20

6-Butyl-1-((2′-(1H-tetrazol-5-yl)[1,1′-biphenyl]-4-yl) methyl)-2(1H)-pyridinone dipotassium salt

a) 4′-((6-Butyl-2-oxo-1,2-dihydropyridin-1-yl)methyl)[1,1′-biphenyl]-2-carbonitrile

6-Butyl-2(1H)-pyridinone (1.36g.,9mmol) in dry dimethyl formamide (10 ml) was added dropwise to a stirred suspension of sodium hydride (280 mg. of 80% suspension in oil, 9mmol) in dry dimethyl formamide (20 ml) under nitrogen. After stirring the solution obtained, at 20° for 30 minutes, 4′-(bromomethyl) [1,1′-biphenyl]-2-carbonitrile (2.72 g, 10mmol) in dry dimethyl formamide (10 ml) was added dropwise. The resulting orange solution was stirred at 20° for 24 hrs, then poured into saturated brine (250 ml) and ethyl acetate (100 ml). The organic phase was separated, dried ($MgSO_4$), and concentrated under vacuum to give an amber syrup (3.1 g). This was separated by chromatography into two components on a silica gel column using ethyl acetate/hexane mixtures as eluant. 4′-((6-Butyl-pyridin-2-yl)oxymethyl)[1,1′-biphenyl]-2-carbonitrile eluted first and was obtained as a viscous oil (2.0 g, 65%). The subtitle compound eluted second and was obtained as pale yellow plates (0.55 g, 18%) mp 109-110° after trituration with diethyl ether.

b) 6-Butyl-1-((2′-(1H-tetrazol-5-yl)[1,1′-biphenyl]-4-yl) methyl)-2(1H)-pyridinone

A solution of the product of step a) (0.5 g, 1.5mmol) and trimethylstannyl azide (0.9g, 4.4mmol) in dry toluene (10 ml) was heated at reflux under nitrogen for 24 hrs. Fresh azide (0.3 g) was then added and heating continued for a further 18 hrs. The solution was cooled and the solvent removed by evaporation under reduced pressure. The residual gum was treated with silica gel (10g) in methanol (100 ml) and the resulting suspension stirred for 30 minutes at room temperature. The methanol was removed by evaporation and the silica and absorbed material applied to the top of a silica column which had been packed in dichloromethane. The chromatogram was developed using dichloromethane/methanol mixtures as eluant and the product-containing fractions were combined and concentrated to leave a colourless foam (0.5 g, 89%). Repeated trituration with dry diethyl ether gave pure title compound as a cream powder mp 193-194° (dec).

c) 6-Butyl-1-{(2′-[1H-tetrazol-5-yl][1,1′-biphenyl]-4-yl) methyl}-2(1H)-pyridinone dipotassium salt

6-Butyl-1-{(2′-[1H-tetrazol-5-yl][1,1′-biphenyl]-4-yl) methyl}-2(1H)-pyridinone (0,050g) was suspended in

methanol (10ml) and 0.1M aqueous potassium carbonate solution (1.246ml).

The mixture was placed in an ultrasonic bath for 15 minutes to give a clear solution, which was partially evaporated and then freeze-dried. The residue was crystallised from acetonitrile/methanol to afford the title dipotassium salt as a solid.

Anal Found C, 55.8; H, 4.7: N, 14.7%

Calc for $C_{23}H_{21}N_5O_2K_2$ 0.76mol $H_2O$; C, 56. 2;
H, 4.6; N, 14.25%

Examples 21 to 23 were prepared using the methods analogous to those of Example 20.

Example 21

2-{4-[(6-Butyl-1,2-dihydro-4-hydroxy-2-oxo-pyridin-1-yl}methyl]napthalene-1-yl}benzoic acid

mp 178-179° ,

Example 22

Phenylmethyl 6-butyl-1,2-dihydro-2-oxo-1-{[2′-(1H-tetrazol-5-yl}[1,1′-biphenyl]-4-yl]methyl}pyridine-3-carboxylate

Example 23

6-Butyl-1,2-dihydro-2-oxo-(N-phenymethyl)-1-{[2′-(1H-tetrazol-5-yl)[1,1′-biphenyl]-4-yl]methyl}pyridine-3-carboxamide

Example 24

6-Butyl-4-hydroxy-1-{[2′-(1H-tetrazol-5-yl)[1,1′-biphenyl]-4-yl]methyl}-2{1H)-pyridinone

a) 4′-{(6-Butyl-1,2-dihydro-4-hydroxy-2-oxopyridine-1-yl) methyl}[1,1′-biphenyl]-2-carbonitrile

A solution of 2-(4′-aminomethyl)phenylbenzonitrile (1.10g) (prepared according to the method of EP 419048) and 6-butyl-4-hydroxy-2-pyrone (1.06g) in aqueous dimethylformamide (10ml, 1:1) was heated at 110° for 20 hours. The reaction was then poured into water and extracted with ethyl acetate. The extracts were washed with water, dried, and evaporated. The residual oil was chromatographed (silica; dichloromethane/methanol 5%) to give the sub-title compound as a pale yellow solid (1.14g). mp 206-207°

b) 6-Butyl-4-hydroxy-1-{[2′-(1H-tetrazol-5-yl][1,1′-biphenyl]-4-yl]methyl}-2(1H)-pyridinone

The nitrile from step a) was reacted with trimethyltin azide by the method of Example 20 b) to give the title compound, mp 239-240°

Anal Found: C,68.85; H5.8; N,17.65%

Calculated for $C_{23}H_{23}N_5O_2$:

C,68.8; H,5.8; N,17.4%

Example 25

4′-[4-Butyl-1,2-dihydro-2-oxopyrimidin-1-yl]-methyl-[1,1′-biphenyl]-2-carboxylic acid

(a) 4-Butyl-2(1H)-pyrimidinone

2-Hydroxy-4-methylpyrimidine hydrochloride (2.2g) was added portionwise to a solution of lithium diisopropylamine (3 equivalents) in dry tetrahydrofuran under nitrogen at -78°. The temperature was allowed to rise to -25° then the solution was recooled to -78° and 1-iodopropane (2.6g) was added in a single portion. The mixture was allowed to warm to room temperature over 16 hours. The mixture was poured into water and acidified to pH5 by addition of 2N hydrochloric acid solution and saturated with sodium chloride. The product was extracted with dichloromethane and the organic phase was washed with brine. Evaporation and chromatography (silica, 3% ethanol/dichloromethane) gave the subtitle product (1.25g),

mp 104-106°

(b) 4′-[4-Butyl-1,2-dihydro-2-oxopyrimidin-1-yl]methyl-[1,1′-biphenyl]-2-carboxylic acid

The product of step (a) was condensed with methyl 4′-bromomethyl[1,1′-biphenyl]-2-carboxylate using the method of Example 1 (a) to furnish the methyl ester of the title product, mp 92-94°. The position of alkylation was established by Nuclear Overhauser effects in the proton NMR spectrum.

The ester was hydrolysed by the method of Example 1 (b) to give the title acid as a gum.
m/z=385(BP, $M^{+}$+1)

Example 26

4′-[(1,2-Dihydro-4-hydroxy-7-methoxy-2-oxo-3-phenylquinolin-1-yl)methyl][1,1′-biphenyl]-2-carboxylic acid

a) Methyl 4′-{[N-(3-methoxyphenyl)-N-trifluoracetylamino] methyl}[1,1′-biphenyl]-2-carboxylate

A solution of 3-methoxy-N-trifluoracetyl aniline (2.2g) in dimethylformamide (20ml) was added to a stirred suspension of sodium hydride (0.26g) in dimethylforamide (15ml). After 2 hours a solution of methyl 4′-bromomethyl [1,1′-biphenyl]-2-carboxylate (3g) in dimethylformamide was added. After 16 hours the solution was poured into brine and extracted with ethyl acetate. The organic phase was washed with brine, dried and evaporated to a brown oil which was chromatographed on silica (petroleum ether, diethyl ether, dichloromethane 80:15:15) to give the sub-titled product as an oil (3.8g).

MS: m/z 443 BP 225

NMR ($CDCl_3$)δH 7.83 (1H,d,ArH), 7.55 (1H, m, ArH), 7.4(1H, m, ArH), 7.33 (1H, m, ArH), 7.25 (5H, m, 5xArH), 6.9 (1H, m, ArH). 6.65 (1H, m, ArH), 6.54 (1H, s, ArH), 4.94 (2H, s, $NCH_2$), 3.73 (3H, s, $CH_3$), 3.65 (3H, s, $CO_2CH_3$),

b) Methyl 4′-[(3-methoxyphenyl)aminomethyl][1,1′-biphenyl]-2-carboxylate

To a solution of the product frog step a) (3.2g) in a mixture of methanol/water 8:1 was added sodium carbonate (10%) aqueous solution. After 16 hours the solution was concentrated, added to water and extracted with ethyl acetate. The organic phase was washed with brine, dried and concentrated to give an oil which was chromatographed on silica (petroleum ether, diethylether, dichloromethane 80:15:5) to give the sub-title product as an oil (2.2g).

M.S: m/z 347 BP 225

NMR ($CDCl_3$)δH 7.83 (1H, m, ArH), 7.5(1H, m, ArH), 7.5 (4H, m, 4xArH), 7.25 (3H, m, 3xArH) 7.1 (1H, t, NH), 6.35 (2H, m, 2xArH), 6.25 (1H, m, ArH), 4.37 (2H, m, $NCH_2$), 3.7 (3H, s, $OCH_3$), 3.65 (3H, s, $CO_2CH_3$),

c) Methyl 4′-[(1,2-dihydro-4-hydroxy-7-methoxy-2-oxo-3-phenylquinolin-1-yl)methyl][1,1′-biphenyl]-2-carboxylate

A mixture of the product from step b) and diethyl phenylmalonate was heated at 220° for 5 hours. The crude product was chromatographed on silica (dichloromethane, ethyl acetate 100:0 - 70:30) to give the subtitle product as a powder (0.8g) m.pt 209-10°.

d) 4′-[(1,2-Dihydro-4-hydroxy-7-methoxy-2-oxo-3-phenylquinolin-1-yl)methyl][1,1′-biphenyl]-2-carboxylic acid

To the product from step c) (0.8g) in methanol/water (5:1) was added 10% sodium hydroxide solution (20ml). After 6 hours under reflux the solution was cooled, concentrated to a small volume and added to water. The mixture was acidified to pH1 and extracted with ethyl acetate. The organic phase was washed with brine, dried and concentrated to give a powder which was recrystallised from ethanol to give the title compound (0.32g).

m.pt 239-41°.

Calc for $C_{30}H_{23}NO_5.0.25H_2O$

C 74.70; H 4.91; N 2.90%

found C 74.53; H 4.87; N 3.15%

Example 27

4′-[(3-Acetyl-1,2-dihydro-4-hydroxy-7-methoxy-2-oxoquinolin-1-yl)methyl][1,1′-biphenyl]-2-carboxylic acid

a) Diethyl 2-(N-({2′-(methoxycarbonyl)[1,1′-biphenyl]-4-yl]methyl)-N-(3-methoxyphenyl))aminocarbonyl-3-oxo-1,5 pentanedioate

Ethyl malonyl chloride (1.2g) in dry dichloromethane (20ml) was added dropwise with stirring to methyl 4′-(3-(methoxyphenyl)aminomethyl[1,1′-biphenyl]-2-carboxylate (2.3g) and pyridine (0.65g) in dry dichloromethane (100ml). After 18 hours further ethyl malonyl chloride (0.6g) and pyridine (0.32g) were added and stirring continued for 4 hours. Water (100ml) was then added and the organic phase was separated, washed with 2M hydrochloric acid, aqueous sodium bicarbonate and brine, then dried and evaporated to a pink oil (3.2g). Chromatography (silica gel/diethyl ether/petroleum ether (40:60) mixtures) gave first the subtitle compound (0.9g) as a colourless syrup (Mass spectrum m/z 576 ($M^{+}$+1)), then ethyl 3-(N-({2′-methoxycarbonyl)[1,1′-biphenyl]-4-yl)methyl)-N-(3-methoxyphenyl)amino)-3-oxopropanoate (1.54g)

b) Methyl 4′-(3,4,5,6-tetrahydro-8-methoxy-2,4,5-trioxo-2H-pyrano[3,2-c]quinolin-6-yl)methyl[1,1′-biphenyl]-2-carboxylate

The sub-title compound from step a) (0.5g) was heated at 220° for 90 minutes. The resulting dark oil was cooled and triturated with ethyl acetate giving the sub-title compound as a pale yellow amorphous powder (150mg),

M.S. m/z 484 ($M^{+}$+1) .

NMR δ(DMSO): 3.30 (2H,s,$COCH_2CO$), 5.70(2H,bs,$NCH_2$) (inter alia)

c) 4′[(3-acetyl-1,2-dihydro-4-hydroxy-7-methoxy-2-oxoquinolin-1-yl)methyl][1,1′-biphenyl]-2-carboxylic acid

The sub-title compound for step b) (120mg) was heated at reflux with sodium hydroxide (100mg) in water (5ml) and methanol (5ml) for 8 hours. The solution was poured into water, acidified with 2M hydrochloric acid and the precipitated product separated, washed with water and dried. Trituration with ethyl acetate gave the title compound (66mg) as a pale yellow solid

m.p. 236-9° (decomp)

M.S m/z 444 ($M^{+}$+1)

NMR δ(DMSO): 2.76 (3H,ss,$COCH_3$) 3.81 (3H,s,$OCH_3$), 5.53 (2H,bs,$NCH_2$) (inter alia)

Example 28

4-Hydroxy-6-propyl-1-{(2′-[1H-tetrazol-5-yl][1,1′-biphenyl]-4-yl)methyl}-2(1H)-pyridinone

a) 4′-{(1,2-Dihydro-4-hydroxy-2-oxo-6-propylpyridin-1-yl) methyl}[1,1′-biphenyl]-2-carbonitrile

The sub-title compound was prepared by the method of Example 24 a) from 4-hydroxy-6-propyl-2-pyrone. The compound was obtained as an oil.

MS: m/z 345 ($M+H^{+}$),

′HNMR (salient features): 0.95 (t, $CH_3$), 1.16 (m, $CH_3\underline{CH_2}$) 2.52 (t, $CH_3CH_2\underline{CH_2}$), 5.39 (br.s, $NCH_2$), 6.09 (s, NC=$\underline{CH}$), 6.25 s, O-C=$\underline{CH}$).

b) 4-Hydroxy-6-propyl-1-{(2′-[1H-tetrazol-5-yl](1,1′-biphenyl]-4-yl)methyl}-2(1H)-pyridinone

By the method of Example 24 b) the product from step a) was converted to the title compound,

mp 265-266° (decomp).

Example 29 was prepared by the methods of Example 1b) and 15.

Example 29

6-Butyl-1-{(2′-carboxy[1,1′-biphenyl]-4-yl)methyl}-1,2-di hydro-2-oxopyridine-4-carboxylic acid

Example 30

4′-[(3-Butyl-1,2-dihydro-4-hydroxy-7-methoxy-2-, oxoquinolin-1-yl)methyl][1,1′-biphenyl]-2-carboxylic acid

a) Methyl 4′-{N-(2-[ethoxycarbonyl]-1-oxohexyl)-N-(3-methoxyphenyl)aminomethyl}[1,1′-biphenyl]-2-carboxylate

To methyl 4′[(3-methoxyphenyl)aminomethyl][1,1′-biphenyl]-2-carboxylate (4.0g) and triethylamine (1.6ml) in dry dichloromethane (200ml) at 0° was added ethyl 2-(chlorocarbonyl)hexanoate (2.4g) dropwise with stirring. The mixture was allowed to warm to room temperature over 6 hours and then added to water. The organic phase was washed with water, dried, and evaporated to yield the sub-title compound (6.4g) as an oil.

b) Methyl 4′-[(3-butyl-1,2-dihydro-4-hydroxy-7-methoxy-2-oxoquinolin-1-yl)methyl][1,1′-biphenyl]-2-carboxylate

The product from step a) (6.4g) was heated at 220-240° for 14 hours. The residue was extracted with hot dichloromethane, which was then evaporated to a gum. The gum was chromatographed (silica gel, dichloromethane/ethyl acetate (3%)) to give a solid which crystallised from toluene to afford the sub-title product (0.45g).

mp 161-164°.

c) 4′-[(3-Butyl-1,2-dihydro-4-hydroxy-7-methoxy-2-oxoquinolin-1-yl)methyl][1,1′-biphenyl]-2-carboxylic acid

The product from step b) was hydrolysed by the method of Example 26 d) to afford the product.

mp 216-217°.

Anal Found C, 71.75; H, 5.8; N, 3.1%

Calc for $C_{28}H_{27}N\,O_5$: C, 72.1; H, 6.0: N, 3.0%

Example 31

4-Hydroxy-7-methoxy-3-phenyl-1-((2′-(1H-tetrazol-5-yl) [1,1′-biphenyl]-4-yl)methyl)-2(1H)-quinolinone

a) 4′-[(3-Methoxyphenyl)aminomethyl)][1,1′-biphenyl]-2-carbonitrile

3-Methoxy-N-trifluoroacetyl-aniline and 4′-bromomethyl [1,1′-biphenyl]-2-carbonitrile were reacted and converted by the method of Example 26 a) and b) to the sub-title product as an oil.

MS: m/z 315 ($M^{+}$+1)

′HNMR (δ $CDCl_3$, salient peaks) 7.75 (d,1H, ArH ortho to CN); 4.4 (s, 2H, $NCH_2$); 4.1 (br s, 1H, NH); 3.75 (s, 3H, $OCH_3$).

b) 4′-{(1,2-Dihydro-4-hydroxy-7-methoxy-2-oxo-3-phenylquinolin-1-yl)methyl}[1,1′-biphenyl]-2-carbonitrile

The product from step a) and diethyl phenylmalonate were reacted by the method of Example 26 c) to give the sub-title product as an oil.

MS: m/z 459 ($M^{+}$1)

′HNMR ( δ $CDCl_3$, salient peaks) 7.79 (d, 1H, quinoline-H5); 6.8 (dd, 1H, quinoline-H6); 6.78 (d, 1H, quinoline-H8); 5.6 (br s, 2H, $NCH_2$); 3.2 (s, 3H, $OCH_3$).

c) 4-Hydroxy-7-methoxy-3-phenyl-1-(2′-[1H-tetrazol-5-yl] [1,1′-biphenyl]-4-yl)methyl}-2(1H)-quinolinone

The nitrile product from step b) (1.0g) and trimethyltin azide (2g) were stirred and refluxed in toluene (150ml) under nitrogen for 16 hours. Trimethyltin azide (2g) was then added and the mixture was refluxed for a further 3 days. On cooling, a solid was obtained which was dissolved in dichloromethane/methanol (1:1) mixture and stirred with silica gel (20g). After 30 minutes the solvent was removed and the residue was placed on the top

of a chromatography column. Elution with dichloromethane/methanol (95:5) was afforded a solid, which was crystallised from ethanol to give the title product (0.38g),

mp 227-211°

Anal Found C, 72.1; H, 4.6; N, 14.0%

Calc for $C_{20}H_{23}N_5O_3$: C, 71.85; H, 4.6; N; 14.2%

Examples 32 and 33 were prepared by the method of Example 26

Example 32

4′-[(7-Ethyl-1,2-dihydro-4-hydroxy-3-phenyl-2-oxoquinolin-1-yl)methyl][1,1′-biphenyl]-2-carboxylic acid

m.pt 229-232°

Example 33

4′[(1,2-Dihydro-7-ethoxy-4-hydroxy-2-oxo-3-phenylquinolin-1-yl)methyl][1,1′-biphenyl]-2-carboxylic acid

m.pt 193-195°

Example 34

7-Ethoxy-4-hydroxy-3-phenyl-1-{(2′-[1H-tetrazol-5-yl][1,1′-biphenyl]-4-yl)methyl}-2(1H)-quinolinone

a) 4′-{(7-Ethoxy-1,2-dihydro-4-hydroxy-2-oxo-3-phenylquinolin-1-yl)methyl}[1,1′-biphenyl]-2-carbonitrile

2-Ethoxy-N-trifluoracetyl aniline was reacted by the methods of Examples 26 a), 26 b) and 26 c) to give the sub-title compound,

mp 119-124°

b) 7-Ethoxy-4-hydroxy-3-phenyl-1-{(2′-[1H-tetrazol-5-yl] [1,1′-biphenyl]-4-yl)methyl}-2(1H)-quinolinone

By the method of Example 31 c) the product from step a) was converted to the title compound.

Example 35 was prepared by the method of Example 30.

Example 35

4′-[(1,2-Dihydro-4-hydroxy-7-methoxy-2-oxoquinolin-1-yl)methyl][1,1′-biphenyl]-2-carboxylic acid

m.pt 303-5° (dec)

Example 36

4′[(6-Butyl-1,2-dihydro-4-methoxy-2-oxopyridin-1-yl) methyl][1,1′-biphenyl]-2-carboxylic acid

a) Methyl 4′-[(6-Butyl-1,2-dihydro-4-methoxy-2-oxopyridin-1-yl)methyl][1,1′-biphenyl]-2-carboxylate

Methyl 4′-[(6-butyl-1,2-dihydro-4-hydroxy-2-oxopyridin-1-yl)methyl][1,1′-biphenyl]-2-carboxylate (0.20g) and potassium carbonate (0.091g) were stirred in a solution of dimethylsulphate (62μ1) in 2-butanone (20ml) at ambient temperature, under nitrogen, for 30 hours. The reaction mixture was poured into water and extracted with ethyl acetate. The extracts were dried, evaporated, and the residual oil purified by column chromatography (silica gel, eluting with 40% ethyl acetate/60% hexane) to give the sub-title compound as an oil (0.19g, 91%).

MS: m/z 405 ($M^+$), 225 (BP).

′HNMR: (δ $CDCl_3$): 3.62 (s, $CO_2CH_3$), 3.79 (s, $OCH_3$), 5.35 (br s, $NCH_2$), 5.81 (s, NC=CH), 5.96 (s, O-C=CH) (inter alia).

b) 4′-[(6-Butyl-1,2-dihydro-4-methoxy-2-oxopyridin-1-yl) methyl[1,1′-biphenyl]-2-carboxylic acid

The ester from step a) was hydrolised by the method of Example 1 b) to furnish the title acid,

mp 119-120°.

Example 37

6-Butyl-4-hydroxy-3-nitro-1-{[2′-(1H-tetrazol-5-yl)[1.1′-biphenyl]-4-yl]methyl}-2(1H)-pyridin-2-one

a) 4′{(6-Butyl-1,2-dihydro-4-hydroxy-3-nitro-2-oxopyridine-1-yl)methyl}[1,1′-biphenyl]-2-carbonitrile

A solution of 4′{(6-butyl-1,2-dihydro-4-hydroxy-2-oxopyridin-1-yl)methyl}[1,1′-biphenyl]-2-carbonitrile (1.00g) in glacial acetic acid (10ml) and concentrated nitric acid (2.0ml of d 1.42) was warmed to 90° for 15 minutes. On cooling the sub-title compound crystallised out and was collected by filtration, washing with water, and drying under vacuum. The sub-title compound was obtained as a yellow solid (0.95g).

mp 126-127° (decomp)

b) 6-Butyl-4-hydroxy-3-nitro-1-{[2′-{1H-tetrazol-5-yl)[1,1′-biphenyl]-4-yl]methyl-2(1H)-pyridinone

The product of step a) was reacted with trimethyltin azide by the method of Example 20 b) to give the title compound,

mp 130-131°.

Example 38

6-Butyl-1,2-dihydro-2-oxo-1-{(2′-[1H-tetrazol-5-yl][1,1-biphenyl]-4-yl)methyl}pyridine-4-carboxylic acid

Ethyl 6-butyl-1,2-dihydro-2-oxo-1-{(2′-[1H-tetrazol-5-yl][1,1′-biphenyl]-4-yl)methyl}pyridine-4-carboxylate was hydrolised according to the method of Example 1 b) to give the title acid.

Example 39

Ethyl 1,2-dihydro-4-hydroxy-3-methyl-2-oxo-6-propyl-1-{(2′-[1H-tetrazol-5-yl][1,1′-biphenyl]-4-yl)methyl}pyridine-5-carboxylate

a) Ethyl {(2′-cyano[1,1′-biphenyl]-4-yl)methyl}-1,2-dihydro-4-hydroxy-3-methyl-2-oxo-6-propylpyridine-5-carboxylate

To methyl malonyl dichloride (9.4g) stirred at 90° in dry toluene (30ml) under nitrogen was added dropwise ethyl 3-oxohexanoate (12g). The mixture was heated at 90° for 18 hours, then cooled and extracted repeatedly with 10% sodium carbonate solution. The aqueous phase was acidified and extracted with diethyl ether, which was washed with brine, dried, and evaporated to an oil.

The oil was chromatorgraphed (silica gel, dichloromethane) to give ethyl 4-hydroxy-3-methyl-2-oxo-6-propyl-(2H)-pyran-5-carboxylate as an oil (2g).

′HNMR ($CDCl_3$, salient peaks) δ 11.6 (s,1H, OH); 4.4 (q, 2H, $CO_2CH_2$); 2.9 (t, 2H, $-CH_2$-pyrone; 2.0 (s, 3H, pyrone-$CH_3$); 1,4 (t, 3H, ester $CH_3$).

The above pyrone (2g) and 4′-amino [1,1′-biphenyl]-2-carbonitrile (1.7g) were refluxed in aqueous dimethyl formamide according to the method of Example 24 a) to give the sub-title compound as a gum.

′HNMR ($CDCl_3$, salient peaks) δ 11.7 (s, 1H, OH); 7.76 (d, 1H, ArH ortho to CN) 5.3 (s, 2H, $NCH_2$); 3.0 (v.br, 2H, pyridine-$CH_2$); 2,1 (s, 3H, pyridine-$CH_3$).

b) Ethyl 1,2-dihydro-4-hydroxy-3-methyl-2-oxo-6-propyl-1-{(2′-[1H-tetrazol-5-yl](1,1′-biphenyl]-4-yl)methyl} pyridine-5-carboxylate

The product of step a) was converted by the method of Example 20 b) to the sub-title tetrazole.

c) 1,2-Dihydro-4-hydroxy-3-methyl-2-oxo-6-propyl-1-{(2′-[1H-tetrazol-5-yl][1,1′-biphenyl]-4-yl)methyl}pyridine-5-carboxylic acid

Ethyl 1,2-dihydro-4-hydroxy-3-methyl-2-oxo-6-propyl-1-((2′-[tetrazol-5-yl][1,1′-biphenyl]-4-yl]methyl)pyridine-5-carboxylate was hydrolysed by the method of Example 1 b) to give the title product.

Example 40

6-Butyl-4-hydroxy-1-[(3-bromo-2-(2-{1H-tetrazol-5-yl) phenyl)benzofuran-5-yl)methyl]-2(1H)-pyridinone

a) 5-(Azidomethyl)-3-bromo-2-(2-(2-(triphenylmethyl)-2H-tetrazol-5-yl)phenyl)-benzo[b]furan

Sodium azide (0.68g, 10.46mmol) was added to 3-bromo-5-(bromomethyl)-2-(2-(2-(triphenylmethyl)-2H-tetrazol-5-yl) phenyl)-benzo[b]furan (prepared according to the method of EP 434249) (5.9g, 8.73mmol) in dry dimethylformamide. The resulting suspension was stirred at 20° for 18 hours, then poured into water (250ml). The precipitate was collected and washed with water and the solid produced extracted with diethyl ether. The extracts were washed with water, dried and concentrated under vacuum leaving a solid which on trituration with a small volume of ice cold diethyl ether gave the subtitle compound as a white powder, (4.7g)

m.p 161-3° (dec.)

b) 3-Bromo-2-(2-(2-(triphenylmethyl)-2H-tetrazol-5-yl)phenyl)benzo[b]furan-5-methanamine

Triphenylphosphine (1.88g, 7.2mmole) was added to a solution of the product of step a) (4.46g, 7 mmole) in dry tetrahydrofuran (40ml) with stirring under nitrogen at 20°. After 24 hours water (0.5ml) was added and stirring continued for 18 hours. The resulting solution was concentrated to dryness under vaccum and the gummy residue was taken up in dichloromethane (100ml), washed with brine, dried and evaporated. Trituration of the foamy residue with cold diethyl ether gave the crude amine as a white amorphous powder m.p 148-151° (3.6g).

c) 6-Butyl-4-hydroxy-1-[(3-bromo-2-(2-(1H-tetrazol-5-yl)phenyl)benzo[b]furan-5-yl)methyl]-2(1H)-pyridinone

A suspension of the product from step b) (1.53g, 2.5mmoles) and 6-butyl-4-hydroxy-2H-pyran-2-one (0.42g, 2.5mmoles) in dimethyformamide (10ml) and water (15ml) was stirred at 125° for 18 hours. The resulting clear, yellow solution was decanted from a tarry residue and cooled in ice. The solid which separated was filtered, washed with water and dried. The solid was taken up in ethyl acetate (50ml) and sodium bicarbonate solution (50ml) and the aqueous phase separated and acidified with 2M hydrochloric acid. The material that separated was extracted into ethyl acetate and the extracts were washed with water and dried.

Evaporation of the solvent gave the title compound (0.2g) as an amorphous powder after trituration with diethyl ether.

mp 190° (decomp).

Examples 41 to 42 were prepared by the methods of Example 40.

Example 41

2-{3-Bromo-5-[(7-ethoxy-1,2-dihydro-4-hydroxy-2-oxo-3-phenylquinolin-1-yl)methyl]benzofuran-2-yl}benzoic acid

Example 42

7-Ethoxy-4-hydroxy-3-phenyl-1-{[3-bromo-2-(2-(1H-tetrazol-5-yl)phenyl)benzofuran-5-yl]methyl}-2(1H)-quinolinone.

Examples 43 to 46 were prepared by the method of Example 7 using the intermediates of WO 91/12001 and intermediates of previous Examples.

Example 43

1-[(3-Bromo-2-(2-(1H-tetrazol-5-yl)phenyl)benzofuran-5-yl)methyl]-4-hydroxy-6-propyl-2(1H)-pyridinone

Example 44

α-{4-{[1,2-Dihydro-4-hydroxy-2-oxo-6-propylpyridin-1-yl]methyl)Phenoxy}benzeneacetic acid

Prepared using intermediates of Example 7.

Example 45

α-{4-([6-Butyl-1,2-dihydro-4-hydroxy-2-oxopyridin-1-yl]methyl)phenoxy)benzeneacetic acid

Example 46

4-Hydroxy-1-{[4-{phenyl[1H-tetrazol-5-yl]methoxy)phenyl]methyl}-6-propyl-2(1H)-pyridinone

Prepared using intermediates of Example 28.

Example 47

6-Butyl-4-hydroxy-1-{[4-(phenyl[1H-tetrazol-5-yl] methoxy)phenyl]methyl}-2{1H}-pyridinone

Prepared using intermediates of Example 7.

Example 48

4′-[(6-Butyl-1,2-dihydro-3-hydroxymethyl-2-oxo-pyridin-1-yl)methyl][1,1′-biphenyl]-2-carboxylic acid

a) Trimethylsilyl 4′-[(6-butyl-1,2-dihydro-3-hydroxymethyl-2-oxopyridin-1-yl)methyl)methyl][1,1′-biphenyl]-2-carboxylate

6-Butyl-1,2-dihydro-2-oxo-1-{(2′-[2-trimethylsilylethoxycarbonyl][1,1′-biphenyl]-4-yl)methyl}pyridine-3-carboxylic acid (1.4g) was dissolved in dimethoxyethane (5ml) under nitrogen and cooled to -15°. Triethylamine (0.43ml) was added followed by isobutylchloroformate (0.4ml) and the suspension was stirred at -15° for 5 minutes, then filtered. The filtrate was recooled to -15° and sodium borohydride (0.16g) in water (1.4ml) was added. The mixture was stirred for 2 minutes, then diluted with water and extracted with ethyl acetate. The organic phase was then washed with brine and dried. Evaporation and chromatography (silica gel; ethanol/dichloromethane/formic acid 1:99:0.1) of the residue gave the subtitle compound (0.85g) as an oil.

′HNMR δ ($CDCl_3$), (salient peaks):

7.25 - 7.3 (m,4H,Ar-H and pyridinone-4H); 6.1 (d, 1H, pyridinone-5H), 4.6 (s,2H $CH_2OH$); 4.15 (m, 2H, $CO_2CH_2$); 0.85 (m, 2H, $CH_2Si$); 0.0 ($SiMe_3$).

b) 4′-[(6-Butyl-1,2-dihydro-3-hydroxymethyl-2-oxo-pyridin-1-yl)methyl][1,1′-biphenyl]-2-carboxylic acid

By the method of Example 8 d), the product of step a) was converted to the title compound,
m.p 156 - 157°

Example 49

3,5-Dibromo-6-butyl-4-hydroxy-1-[(2′-(1H-tetrazol-5-yl)[1,1′-biphenyl]-4-yl)methyl]-2(1H)-pyridinone

a) 4′-{(3,5-Dibromo-6-butyl-1,2-dihydro-4-hydroxy-2-oxopyridin-1-yl)methyl}[1,1′-biphenyl]-2-carbonitrile

4′-{(6-Butyl-1,2-dihydroxy-4-hydroxy-2-oxo-pyridin-1-yl)methyl}[1,1′-biphenyl]-2-carbonitrile (1g) was suspended in water (10ml). Bromine (0.32ml) was added and the mixture was stirred at room temperature for 2 hours. The water was removed by evaporation and coevaporation with ethanol to yield a solid which was triturated with diethyl ether. The solid was extracted with warm methanol, which was filtered and evaporated to yield the sub-title nitrile (0.65g)
m.p 198° (dec)

b) 3,5-Dibromo-6-butyl-4-hydroxy-1-[(2′-(1H-tetrazol-5-yl)[1,1-biphenyl]-4-yl)methyl]-2(1H)-pyridinone

By the method of Example 20 b), the product of step a) was converted to the title compound,
m.p 212 - 215° (dec)

Example 50

3-Bromo-6-butyl-4-hydroxy-1-[(2′-(1H-tetrazol-5-yl)[1,1′-biphenyl]-4-yl)methyl]-2(1H)-pyridinone

a) 4′-{(3-Bromo-6-butyl-1,2-dihydro-4-hydroxy-2-oxopyridin-1-yl)methyl}[1,1′-biphenyl]-2-carbonitrile

4′-{(6-Butyl-1,2-dihydro-4-hydroxy-2-oxo-pyridin-1-yl)methyl)[1,1′-biphenyl]-2-carbonitrile (0.8g) was stirred in dichloromethane (30ml) and methanol (1ml). N-Bromosuccinimide (0.4g) was added and the mixture was stirred at room temperature for 20 minutes. The resulting suspension was filtered to yield the sub-title compound as a solid (0.83g)

m.p 246 - 247° (dec)

b) 3-Bromo-6-butyl-4-hydroxy-1-[(2′-(1H-tetrazol-5-yl)[1,1′-biphenyl]-4-yl)methyl]-2(1H)-pyridinone

By the method of Example 20 b), the product of step a) was converted to the title compound.

Example 51

Ethyl 6-butyl-1,2-dihydro-2-oxo-1-{(2′-[1H-tetrazol-5-yl][1,1′-biphenyl]-4-yl)methyl}pyridine-4-carboxylate

a) Ethyl 6-butyl-1,2-dihydro-2-oxo-1-({2′-(triphenylmethyl)-[1H-tetrazol-5-yl][1,1′-biphenyl]-4-yl}methyl)pyridine-4-carboxylate

Ethyl 6-butyl-1,2-dihydro-2-oxopyridine-4-carboxylate (mp 101-102°) was prepared by esterification of the corresponding acid and ethanol and concentrated sulphuric acid. This ester (1.56g) in dry dimethyl formamide (5ml) was added to a stirred suspension of sodium hydride (60% disp; 0.34g, 8.5MMol) in dimethyl formamide (10ml). After 30 minutes a solution of 4-bromomethyl-2′-[(2-(triphenylmethyl)-[1H-tetrazol-5-yl][1,1′-biphenyl] (3.9g,) in dimethylformamide (30ml) was added and stirring continued for 12 hours. Water (100ml) was added and the solution extracted with ethylacetate which was washed with brine, dried and evaporated. Purification of the residue by chromatography (95:5 dichloromethane:methanol) gave the sub-title compound.

b) Ethyl 6-butyl-1,2-dihydro-2-oxo-1-{(2′-[1H-tetrazol-5-yl][1,1′-biphenyl]-4-yl)methyl}pyridine-4-carboxylate

The product from step a) was stirred in ethanol (80ml) containing concentrated hydrochloric acid (0.8ml) for 16 hours. The mixture was evaporated, and the residue was extracted with ethyl acetate, which was washed with brine, dried, and evaporated to give the title ester.

Example 52

4-Hydroxy-7-methylthio-3-phenyl-1-{(2′-(1H-tetrazol-5-yl][1,1′-biphenyl]-4-yl)methyl}-2(1H)-quinolinone

a) 4′-[(3-methylthiophenyl)aminomethyl][1.1′-biphenyl]-2-carbonitrile

3-Methylthio-N-trifluoroacetylaniline was prepared and reacted with 4′-bromomethyl[1,1′-biphenyl]-2-carbonitrile by the method of Example 26 a) to give the N-trifluoroacetyl derivative of the sub-title product as an oil. Hydrolysis by the method of Example 26 b) gave the sub-title nitrile as an oil.

MS: m/z 331 ($M^{+}1$)

′HNMR ( δ DMSO, salient peaks) 7.0 (t, 1H, ArH meta to $CH_3S$), 6.51 (s, 1H, ArH ortho to $CH_3S$ and NH), 4.36 (d, 2H, N-$CH_2$), 2.36 (s, 3H, $CH_3$).

b) 4′-{(1,2-Dihydro-4-hydroxy-7-methylthio-2-oxo-3-phenylquinolin-1-yl)methyl}[1,1′-bipheny]-2-carbonitrile

The product from step a) (4.7g) and diethylphenylmalonate (3.4g) were heated as a melt at 220° with stirring for 5 hours as in the method of Example 26 c) to give the sub-title nitrile as an oil (0.39g).

MS: m/z 475 ($M^{+}1$)

′HNMR ( δ DMSO, salient peaks) 10.26 (s, 1H, OH), 5.62 (br s, 2H, $NCH_2$), 2.45 (s 3H, $CH_3S$).

c) 4-Hydroxy-7-methylthio-3-phenyl-1-{(2′-[1H-tetrazol-5-yl][1,1-biphenyl]-4-yl)methyl}-2(1H)-quinolinone

By the method of Example 31 c), the product from step b) was refluxed in toluene with trimethyltin azide to give the title compound.

mp 211°.

Example 53

4-Hydroxy-7-methylsulphonyl-3-phenyl-1-{(2′-[1H-tetrazol-5-yl][1,1′-biphenyl]-4-yl)methyl}-2(1H)-quinolinone

To 4-hydroxy-7-methylthio-3-phenyl-1-{(2′-[1H-tetrazol-5-yl][1,1′-biphenyl]-4-yl)methyl}-2(1H)-quinolinone of Example 52 (0.6g) in methanol (20ml) was added potassium peroxymonosulphate (1.95g) in water (20ml). The mixture was refluxed for 2 hours, cooled and then partitioned between ethyl acetate and saturated aqueous sodium hydrogen sulphite. The organic layer was washed with brine, dried, and evaporated to a residue which was crystallised from a small volume of ethanol to afford the title sulphone, (0.1g), mp 214°

MS: m/z 550 ($M^{+}$+1)

'HNMR ( δ DMSO, salient peaks): 5.58 (s, 2H, $NCH_2$); 3.21 (s, 3H, $SO_2CH_3$).

Example 54

6-Butyl-4-methoxy-1-{[2′-(1H-tetrazol-5-yl)[1,1′-biphenyl]-4-yl]methyl}-2(1H)-pyridinone

a) 4′-{(6-Butyl-1,2-dihydro-4-methoxy-2-oxopyridin-1-yl)methyl}[1,1′-biphenyl]-2-carbonitrile

The sub-title compound was prepared by the method of Example 36 from 4′{(6-butyl-1,2-dihydro-4-hydroxy-2-oxopyridin-1-yl)methyl}[1,1′-biphenyl]-2-carbonitrile, and was obtained as an oil.

MS: m/z 373 (M+$H^{+}$, BP).

'HNMR: (salient peaks): δ 3.81 (s, $OCH_3$), 5.37 (br s $NCH_2$), 5.86 (s, NC=C<u>H</u>), 6.02 (s, O-C=C<u>H</u>).

b) 6-Butyl-4-methoxy-1-{[2′-(1H-tetrazol-5-yl)[1,1′-biphenyl]-4-yl]methyl}-2(1H)-pyridinone

By the method of Example 20 b) the product of step a) was converted to the title product,

mp 191 - 191.5°

Example 55

4′-[(6-Butyl-1,2-dihydro-4-hydroxy-3-nitro-2-oxopyridin-1-yl)methyl][1,1′-biphenyl]-2-carboxylic acid

Methyl 4′-[(6-butyl-1,2-dihydro-4-hydroxy-2-oxopyridin-1-yl)methyl][1,1′-biphenyl]-2-carboxylate was nitrated by the method of Example 37. The reaction mixture was diluted with water to afford the methyl ester of the title acid.

MS: m/z 437 (M+$H^{+}$), 225 (BP)

'HNMR : (salient peaks) δ 3.65 (s, $CO_2CH_3$), 5.26 (br.s , $NCH_2$), 6.00 (s, pyr-H5), 13.05 (br.s, OH).

Hydrolysis of the ester by the method of Example 1 b) gave the title acid,

mp 130-131°.

Example 56

4-Hydroxy-7-methyl-3-phenyl-1-((2′-(1H-tetrazol-5-yl) [1,1′-biphenyl]-4-yl)methyl)-2(1H)-naphthyridone

a) Methyl 2-{(2′-cyano[1,1′-biphenyl]-4-yl)methyl}amino-6-methylpyridine-3-carboxylate

Methyl 6-methyl-2-chloropyridine-3-carboxylate (6.84g), and 4′-aminomethyl[1-1′-biphenyl]-2-carbonitrile 7.67g), in acetonitrile were heated at 100° for 16 hours. After cooling, water was added and the solution was extracted with ethyl acetate. The combined organics were washed with brine, dried and evaporated. Purification of the residue by chromatography gave the sub-title compound.

m/z 358 ($M^{+}$)

'HNMR ($CDCl_3$, salient peaks) δ 8.01 (d, 1H, pyridine-H4); 6.45 (d, 1H, pyridine-H5); 4.87 (d, 2H, $NCH_2$);

3.87 (s, 3H, $CO_2CH_3$); 2.43 (S, 3H, pyridine-$CH_3$)

b) 4-hydroxy-7-methyl-3-phenyl-1-((2′-(1H-tetrazol-5-yl) [1,1′-biphenyl]-4-yl)methyl)-2(1H)-naphthyridone

The product from step a) was reacted with phenylacetyl chloride in dichloromethane containing triethylamine to give methyl 2-{N-[(2′-cyano[1,1′-biphenyl]-4-yl)methyl]-N-(phenylacetyl)amino-6-methylpyridine-3-carboxylate, which was treated with one equivalent of sodium methoxide in methanol to afford 4′-{(1,2-dihydro-4-hydroxy-7-methyl-2-oxo-3-phenyl naphthyridin-1-yl)methyl)[1,1′-biphenyl]-2-carbonitrile.

This was converted by the method of Example 31 c) to give the title product.

Example 57

4′-[(3-Acetylamino-6-butyl-1,2-dihydro-4-hydroxy-2-oxopyridin-1-yl)methyl][1,1′-biphenyl]-2-carboxylic acid

A suspension of 4′-[(6-butyl-1,2-dihydro-3-nitro-4-hydroxy-2-oxopyridin-1-yl)methyl][1,1′-biphenyl]-2-carboxylic acid (0.58g) and iron powder (0.23g) in glacial acetic acid (25ml) was heated at reflux temperature for 1 hour. The reaction was then poured into water and extracted with ethyl acetate. The extracts were washed with water and brine, dried, and the solvent evaporated. The crude product was chromatographed (silica gel; eluting with 2% ethanol in dichloromethane) and then recrystallised from ethyl acetate/cyclohexane to give the title compound (0.50g).

mp 217-218°.

Example A

In vitro Assay of inhibitors of Angiotensin II Co-binding

Potential antagonists of angiotensin II were identified by their ability to inhibit the binding of $^3$H-AII to receptors in rat adrenal cortical microsomes, as described by Glossman et al in J Biol Chem 1974, 249, 825.

Example B

Antagonism of myotropic effects of Angiotensin II

The antagonistic effects of the test compounds were determined by the degree of inhibition of contractions of isolated rabbit aorta, maintained in a physiological salt solution at 37°, induced by accumulative doses of Angiotensin II.

Example C

In vivo potency and duration in conscious rats

The effects of the test compounds dosed parentally or orally to conscious normotensive rats were evaluated in procedures similar to those described by Wong et al, J Pharm. Exp. Ther., 259 (2), 861, (1991), except that the rats in groups were anaesthetised at given times and their blood pressure increases in response to intravenous Angiotensin II were determined.

Example D

Antihypertensive effects were investigated in conscious spontaneously hypertensive rats (SHR) of the Okamoto strain, as described in Carr et al, Brit. J. Pharmacol. (1990), 100, 83. Experiments were conducted either with or without pretreatment orally with hydrochlorothiazide diuretic. Systolic blood pressure and heart rate were measured by the tail cuff method using an electro-sphygmomanometer 1 hour before and 1, 3, 5 and 24 hours and other times up to 48 hours after oral dosing with the compound (dose range 0.1 - 100mg/kg p.o.). Percentage changes in each parameter were measured with respect to pretreated control values.

Example E

Inhibition of Pressor effects in dogs

Anti-hypertensive effects of the test compounds, dosed either parenterally or orally, were investigated in conscious, catheterised dogs, essentially as described by Carr et al, Brit. J. Pharmacol., (1990), 100, 83. Inhibition of the pressor responses to intravenous Angiotensin II was determined.

Example F

Anxiolytic Activity

Anxiolytic activities of the test compounds compared, to diazepam as a standard, were demonstrated using rats in an elevated plus-maze, in the shape of a cross, with two open arms and two enclosed arms. Decreased preference for the closed arms was assessed, using different doses of the compounds.

## Claims

**1.** A Compound of formula I,

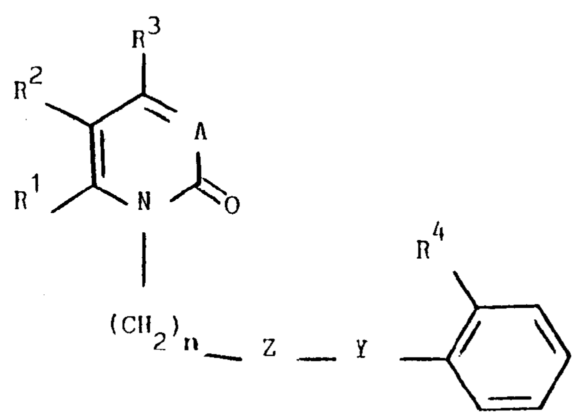

I

In which A is -N- or -$CR^5$-,

$R^2$ is hydrogen, alkyl C1 to 6, halogen or -$COOR^{21}$, or together $R^1$ and $R^2$ form a chain -B=$CR^7$-$CR^8$=$CR^9$-, in which B is in the 8- position,

B is -N- or -$CR^6$-,

$R^6$, $R^7$, $R^8$ and $R^9$, which may be the same or different, are each hydrogen, alkyl C1 to 6, alkoxy C1 to 6, -$S(O)_qR^{22}$ or -$COOR^{23}$,

$R^3$ is hydrogen, hydroxy, alkyl C1 to 6, alkoxy C1 to 6, -$(CH_2)_rCOOR^{10}$,-$(CH_2)_tOR^{31}$ or -$NR^{26}R^{27}$.

$R^5$ is hydrogen, alkyl C1 to 6, alkanoyl C2 to 7, phenyl, halogen, nitrile, nitro, -$NR^{24}R^{25}$, -$CONR^{11}R^{12}$, -$(CH_2)_mOR^{13}$ or -$COOR^{14}$,

Z is a group of formulae II or III

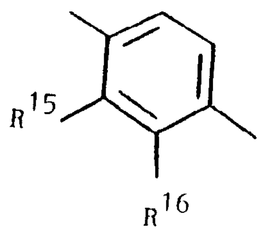

or

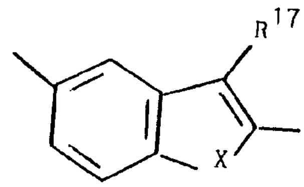

II

III

X is -O-, -S- or $-NR^{18}-$,
$R^{15}$ and $R^{16}$ are each hydrogen or together form a chain -CH=CH-CH=CH-,
$R^{17}$ is hydrogen, halogen, alkyl C1 to 6 or nitrile,
Y is $-(CH_2)_s-$, $-OCHR^{20}-$, $-SCHR^{20}-$, or

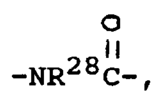

one of $R^4$ and $R^{20}$ is -COOH or tetrazolyl and the remainder is hydrogen,

$R^{12}$, $R^{24}$ and $R^{27}$, which may be the same or different, are each hydrogen, alkyl C1 to 6, alkanoyl C2 to 7, phenyl, $-(CH_2)_pCOOR^{30}$ or phenylalkyl C7 to 12,

$R^{10}$ $R^{14}$ and $R^{30}$ which may be the same or different, are each hydrogen, alkyl C1 to 6, phenyl, phenylalkyl C7 to 12 or $-(CH_2)_lCH(phenyl)_2$,

$R^{11}$, $R^{13}$, $R^{18}$, $R^{21}$, $R^{23}$, $R^{25}$, $R^{28}$, $R^{31}$ and $R^{32}$, which may be the same or different, are each hydrogen or alkyl C1 to 6, or $R^{11}$ and $R^{12}$ may together form a group $-CH_2CH_2MCH_2CH_2-$,

$R^{26}$ is hydrogen, alkyl C1 to 6 or

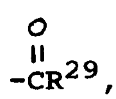

$R^1$, $R^{22}$ and $R^{29}$, which may be the same or different, are each alkyl C1 to 6,

M is -O- or $-NR^{32}-$,

n, m and p, which may be the same or different, are each an integer from 1 to 6, provided that when $R^{27}$ is $-(CH_2)_pCOOR^{30}$ p may be O,

l, r, s and t, which may be the same or different, are each an integer from 0 to 6, and

q is O, 1 or 2,

and pharmaceutically acceptable salts, esters, amides, tautomers and enantiomers thereof.

2. A compound according to Claim 1, wherein
A is $-CR^5-$,
n is 1,
Z is a group of formula II,
Y is a bond and,
$R^1$, $R^2$, $R^3$, $R^4$ and $R^5$ are as defined in Claim 1.

3. A compound according to Claim 1 wherein
A is $-CR^5-$,

$R^1$ is alkyl C1 to 6,
$R^2$ is hydrogen, alkyl C 1 to 6, halogen or $-COOR^{21}$ and
Z, Y, $R^3$, $R^4$, $R^5$ and n are as defined in Claim 1.

**4.** A compound according to Claim 1, wherein
A is $-CR^5-$,
$R^1$ and $R^2$ form a group $-CR^6=CR^7-CR^8=CR^9-$,
$R^7$ is hydrogen, alkyl C1 to 6, alkoxy C1 to 6,
$S(O)_qR_{22}$ or $-COOR^{23}$,
$R^6$, $R^8$ and $R^9$ are each hydrogen, and
Z, Y, $R^3$, $R^4$, $R^5$ and n are as defined in Claim 1.

**5.** A compound according to Claim 1, wherein
A is $-CR^5-$,
$R^1$ is alkyl C1 to 6,
$R^2$ and $R^5$ are each hydrogen,
$R^3$ is hydroxy, alkoxy C1 to 6 or $-(CH_2)_rCOOR^{10}$,
n is 1,
Z is a group of formula **II**,
Y is a bond, and
$R^4$, $R^{10}$ and r are as defined in Claim 1.

**6.** 4′-[(6-Butyl-3-cyano-1,2-dihydro-2-oxo-pyridin-1-yl)methyl]-[1,1′-biphenyl]-2-carboxylic acid dicyclohexylamine salt,
4′-[(3-Cyano-1,2-dihydro-6-methyl-2-oxo-pyridin-1-yl)methyl][1,1′-biphenyl]-2-carboxylic acid,
4′-[3-Cyano-1,2-dihydro-6-methyl-2-oxo-5-propylpyridin-1-yl]methyl[1,1′-biphenyl]-2-carboxylic acid,
6-Butyl-1-[(2′-carboxy[1,1′-biphenyl]-4-yl)methyl]-1,2-dihydro-2-oxopyridine-3-carboxylic acid,
4′-[(6-Butyl-1,2-dihydro-2-oxo-pyridin-1-yl)methyl][1,1′biphenyl]-2-carboxylic acid,
2-{4-[(6-Butyl-1,2-dihydro-2-oxo-pyridin-1-yl)methyl]naphthalene-1-yl}benzoic acid, sodium salt,
4′-[6-Butyl-1,2-dihydro-4-hydroxy-2-oxopyridine-1-yl]methyl[1,1′-biphenyl]-2-carboxylic acid,
Benzyl 6-butyl-1-[(2′-carboxy[1,1′-biphenyl]-4-yl)methyl]-1,2-dihydro-2-oxopyridine-3-carboxylate sodium salt,
Methyl 6-butyl-1-[(2′-carboxy[1,1′-biphenyl]-4-yl)methyl]-1,2-dihydro-2-oxopyridine-3-carboxylate dicyclohexylamine salt,
4′-[(6-Butyl-1,2-dihydro-3-((phenylmethoxy)carbonylmethyl)aminocarbonyl-2-oxo-pyridin-1-yl)methyl][1,1′-biphenyl]-2-carboxylic acid,
4′-[(6-Butyl-1,2-dihydro-3-phenylmethylaminocarbonyl-2-oxo-pyridin-1-yl)methyl][1,1′-biphenyl]-2-carboxylic acid,
4′-[(6-Butyl-1,2-dihydro-3-(N-methoxycarbonylmethyl-N-methylamino)carbonyl-2-oxo-pyridin-1-yl)methyl][1,1′-biphenyl]-2-carboxylic acid,
4′-[(6-Butyl-1,2-dihydro-3-(4-morpholinyl)carbonyl-2-oxo-pyridin-1-yl)methyl][1,1′-biphenyl]-2-carboxylic acid,
4′-[(6-Butyl-1,2-dihydro-3-(4-methyl-1-piperazinyl)carbonyl-2-oxo-pyridin-1-yl)methyl][1,1′-biphenyl]-2-carboxylic acid,
Ethyl 6-butyl-1-[2′-carboxy-[1,1′-biphenyl]-4-ylmethyl]-1,2-dihydro-2-oxo-pyridine-4-carboxylate,
4′-[(6-Butyl-1,2-dihydro-3-(diphenylmethoxy)carbonyl-2-oxo-pyridin-1-yl)methyl][1,1′-biphenyl]-2-carboxylic acid,
4′-[(6-Butyl-3-(carboxymethyl)aminocarbonyl-1,2-dihydro-2-oxo-pyridin-1-yl)methyl][1,1′-biphenyl]-2-carboxylic acid,
4′-[(6-Butyl-3-(2-carboxyethyl)aminocarbonyl-1,2-dihydro-2-oxo-pyridin-1-yl)methyl][1,1′-biphenyl]-2-carboxylic acid,
4′-[(6-Butyl-3-(N-carboxymethyl-N-methyl)aminocarbonyl-1,2-dihydro-2-oxo-pyridin-1-yl)methyl][1,1′-biphenyl]-2-carboxylic acid,
6-Butyl-1-((2′-(1H-tetrazol-5-yl)[1,1′-biphenyl]-4-yl)methyl)-2(1H)-pyridinone dipotassium salt,
2-{4-[(6-Butyl-1,2-dihydro-4-hydroxy-2-oxo-pyridin-1-yl)methyl]napthalene-1-yl}benzoic acid,
Phenylmethyl 6-butyl-1,2-dihydro-2-oxo-1-{[2′-(1H-tetrazol-5-yl)[1,1′-biphenyl]-4-yl]methyl}pyridine-3-carboxylate,
6-Butyl-1,2-dihydro-2-oxo-(N-phenymethyl)-1-{[2-(1H-tetrazol-5-yl)[1,1′-biphenyl]-4-yl]methyl}pyridine-3-carboxamide,

6-Butyl-4-hydroxy-1-{[2 '-(1H-tetrazol-5-yl)[1,1'-biphenyl]-4-yl]methyl}-2(1H)-pyridinone,

4'-[4-Butyl-1,2-dihydro-2-oxopyrimidin-1-yl]-methyl-[1,1'-biphenyl]-2-carboxylic acid,

4'-[(1,2-Dihydro-4-hydroxy-7-methoxy-2-oxo-3-phenylquinolin-1-yl)methyl][1,1'-biphenyl]-2-carboxylic acid,

4'-[(3-Acetyl-1,2-dihydro-4-hydroxy-7-methoxy-2-oxoquinolin-1-yl)methyl][1,1'biphenyl]-2-carboxylic acid,

4-Hydroxy-6-propyl-1-{(2'-[1H-tetrazol-5-yl][1,1'-biphenyl]-4-yl)methyl}-2(1H)-pyridinone,

6-Butyl-1-{(2'-carboxy[1,1'-biphenyl]-4-yl)methyl}-1,2-di hydro-2-oxopyridine-4-carboxylic acid,

4'-[(3-Butyl-1,2-dihydro-4-hydroxy-7-methoxy-2-oxoquinolin-1-yl)methyl][1,1'-biphenyl]-2-carboxylic acid,

4-Hydroxy-7-methoxy-3-phenyl-1-((2'-(1H-tetrazol-5-yl)[1,1'-biphenyl]-4-yl)methyl)-2(1H)-quinolinone,

4'-[(7-Ethyl-1,2-dihydro-4-hydroxy-3-phenyl-2-oxoquinolin-1-yl)methyl][1,1'-biphenyl]-2-carboxylic acid,

4'-[(1,2-Dihydro-7-ethoxy-4-hydroxy-2-oxo-3-phenylquinolin-1-yl)methyl][1,1'-biphenyl]-2-carboxylic acid,

4'-[(1,2-Dihydro-4-hydroxy-7-methoxy-2-oxoquinolin-1-yl)methyl][1,1'-biphenyl]-2-carboxylic acid,

4'-[(6-Butyl-1,2-dihydro-4-methoxy-2-oxopyridin-1-yl)methyl][1,1'-biphenyl]-2-carboxylic acid,

6-Butyl-1,2-dihydro-4-hydroxy-3-nitro-1-{[2'-(1H-tetrazol-5-yl)[1,1'-biphenyl]-4-yl]methyl}pyridin-2-one,

6-Butyl-1,2-dihydro-2-oxo-1-{(2'-[1H-tetrazol-5-yl][1,1biphenyl]-4-yl)methyl}pyridine-4-carboxylic acid,

Ethyl 1,2-dihydro-4-hydroxy-3-methyl-2-oxo-6-propyl-1-{(2'-[1H-tetrazol-5-yl][1,1'-biphenyl]-4-yl)methyl}pyridine-5-carboxylate,

6-Butyl-4-hydroxy-1-[(3-bromo-2-(2-(1H-tetrazol-5-yl)phenyl)benzofuran-5-yl)methyl]-2(1H)-pyridinone,

2-{3-Bromo-5-[(7-ethoxy-1,2-dihydro-4-hydroxy-2-oxo-3-phenylquinolin-1-yl)methyl]benzofuran-2-yl} benzoic acid,

7-Ethoxy-4-hydroxy-3-phenyl-1-{[3-bromo-2-(2-(1H-tetrazol-5-yl)phenyl)benzofuran-5-yl]methyl}-2(1H)-quinolinone,

1-[(3-Bromo-2-(2-(1H-tetrazol-5-yl)phenyl)benzofuran-5-yl)methyl]-4-hydroxy-6-propyl-2(1H)-pyridinone,

α-{4-([1,2-Dihydro-4-hydroxy-2-oxo-6-propylpyridin-1-yl]methyl)phenoxy}benzeneacetic acid,

α-{4-([6-Butyl-1,2-dihydro-4-hydroxy-2-oxopyridin-1-yl]methyl)phenoxy}benzeneacetic acid,

4-Hydroxy-1-([4-(phenyl[1H-tetrazol-5-yl]methoxy)phenyl]methyl)-6-propyl-2(1H)-pyridinone,

6-Butyl-4-hydroxy-1-{[4-(phenyl[1H-tetrazol-5-yl]methoxy)phenyl]methyl}-2(1H)-pyridinone,

4'-[(6-Butyl-1,2-dihydro-3-hydroxymethyl-2-oxo-pyridin-1-yl)methyl][1,1'-biphenyl]-2-carboxylic acid,

3,5-Dibromo-6-butyl-4-hydroxy-1-[(2'-(1H-tetrazol-5-yl)[1,1'-biphenyl]-4-yl)methyl]-2(1H)-pyridinone,

3-Bromo-6-butyl-4-hydroxy-1-[(2'-(1H-tetrazol-5-yl)[1,1'-biphenyl]-4-yl)methyl]-2(1H)-pyridinone,

Ethyl 6-butyl-1,2-dihydro-2-oxo-1-{(2'-[1H-tetrazol-5-yl][1,1'-biphenyl]-4-yl)methyl}pyridine-4-carboxylate,

4-Hydroxy-7-methylthio-3-phenyl-1-{(2'-[1H-tetrazol-5-yl][1,1'-biphenyl]-4-yl)methyl}-2(1H)-quinolinone,

4-Hydroxy-7-methylsulphonyl-3-phenyl-1-{(2'-[1H-tetrazol-5-yl][1,1'-biphenyl]-4-yl)methyl}-2(1H)-quinolinone,

6-Butyl-4-methoxy-1-{[2'-(1H-tetrazol-5-yl)[1,1'-biphenyl]-4-]methyl}-2(1H)-pyridinone,

4'-[(6-Butyl-1,2-dihydro-4-hydroxy-3-nitro-2-oxo-pyridin-1-yl)methyl][1,1'-biphenyl]-2-carboxylic acid,

7-Ethoxy-4-hydroxy-3-phenyl-1-{(2'-[1H-tetrazol-5-yl[1,1'-biphenyl]-4-yl)methyl)-2(1H)-quinolinone,

4-Hydroxy-7-methyl-3-phenyl-1-((2'-(1H-tetrazol-5-yl)[1,1'-biphenyl]-4-yl)methyl)-2(1H)-naphthyridinone.

4'-[(3-Acetylamino-6-butyl-1,2-dihydro-4-hydroxy-2-oxopyridin-1-yl)methyl][1,1'-biphenyl]-2-carboxylic acid,

or a pharmaceutically acceptable salt, ester, enantiomer or tautomer thereof.

7. The use of a compound of formula I or a pharmaceutically acceptable salt, ester, enantiomer or tautomer thereof as a pharmaceutical.

8. A pharmaceutical formulation comprising a therapeutically effective amount of a compound of formula I,

or a pharmaceutically acceptable salt, ester, enantiomer or tautomer thereof, in admixture with a pharmaceutically acceptable adjuvant, diluent or carrier.

9. A process for the production of a compound of formula I or a pharmaceutically acceptable salt, ester, amide, enantiomer or tautomer thereof, which comprises
   a) removal of a protecting group from a compound of formula I in which one or more of the amino hydroxy, carboxylic acid or tetrazolyl groups is protected,
   b) preparation of a compound of formula I which comprises reacting a compound of formula IV,

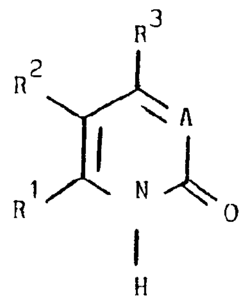

IV

which A, $R^1$, $R^2$ and $R^3$ are as defined above,
with a compound of formula V

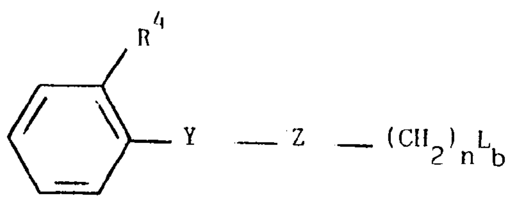

V

in which Y, Z, $R^4$ and n are as defined above, and
$L_b$ is a leaving group
c) preparation of a compound of formula I in which
A is $-CR^5-$,
$R^1$ is alkyl C1 to 6,
$R^2$ is hydrogen, alkyl C1 to 6 or $-COOR^{21}$,
$R^3$ is hydroxy, and
$R^5$ is hydrogen, alkyl C1 to 6, phenyl or $-(CH_2)_mOR^{13}$, which comprises reacting a compound of formula VI,

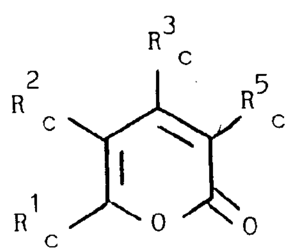

VI

in which $R^1_c$ is alkyl C1 to 6,
$R^2_c$ is hydrogen, alkyl C1 to 6 or $-COOR^{21}$
$R^3_c$ is hydroxy, and
$R^5_c$ is hydrogen, alkyl C1 to 6, phenyl or $-(CH_2)_mOR^{13}$,

with a compound of formula VII,

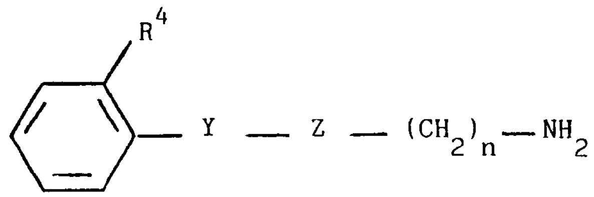

VII

in which Y, Z, $R^4$ and n are as defined above,

d) preparation of a compound of formula I in which A is $-CR^5-$,

$R^3$ is hydroxy,

$R^1$ and $R^2$ together form a chain $-CR^6=CR^7-CR^8=CR^9$, and

$R^5$ is hydrogen, alkyl C1 to 6, phenyl, $-NR^{24}R^{25}$ or $-(CH_2)_mOR^{13}$,

which comprises, heating a compound of formula XV,

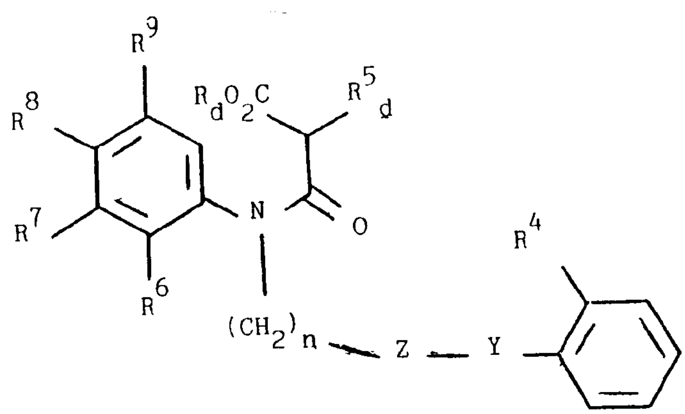

XV

in which Z, Y, n, $R^4$, $R^5$, $R^6$, $R^7$, $R^8$ and $R^9$ are as defined above,

$R^5_d$ is hydrogen, alkyl C1 to 6, phenyl $-NR^{24}R^{25}$ or $-(CH_2)_mOR^{13}$, and

$R_d$ is alkyl C1 to 6,

e) preparation of a compound of formula I in which at least one of $R^3$ or $R^5$ is amino, hydroxy or hydroxyalkyl C1 to 6, which comprises reducing a compound of formula I in which at least one of $R^3$ and $R^5$ contains a group $-NO_2$, -COOH, $-OR_e$ or alkyl $OR_e$,

wherein $R_e$ is alkyl,

f) preparation of a compound of formula I in which $R^7$ is $-S(O)_qR^{22}-$ and q is 1 or 2, by oxidising a compound of formula I in which q is o,

g) preparation of a compound of formula I in which at least one of $R^2$ and $R^5$ represents halogen, which comprises halogenating a compound of formula I in which at least one of $R^2$ and $R^5$ is hydrogen,

h) preparation of a compound of formula I in which $R^5$ is $-COCH_3$, which comprises hydrolysing a compound of formula XII

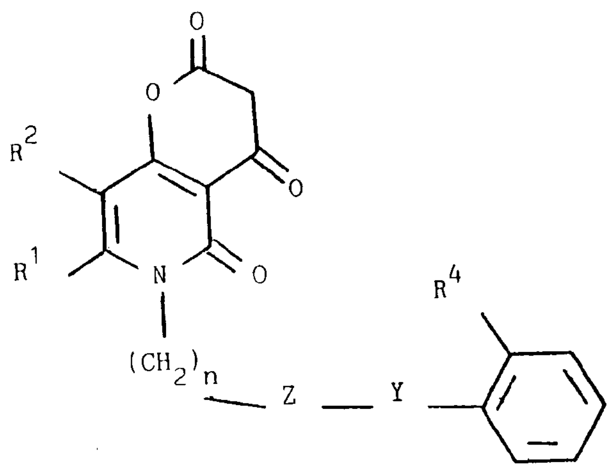

XII

i) preparation of a compound of formula I in which $R^4$ or $R^{20}$ is tetrazolyl, which comprises reacting a compound of VIII or IX,

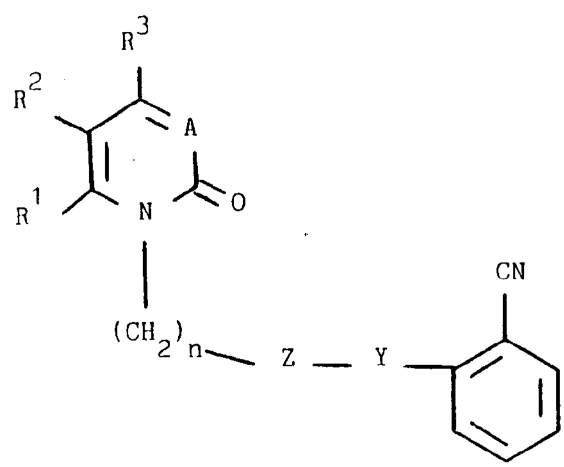

VIII

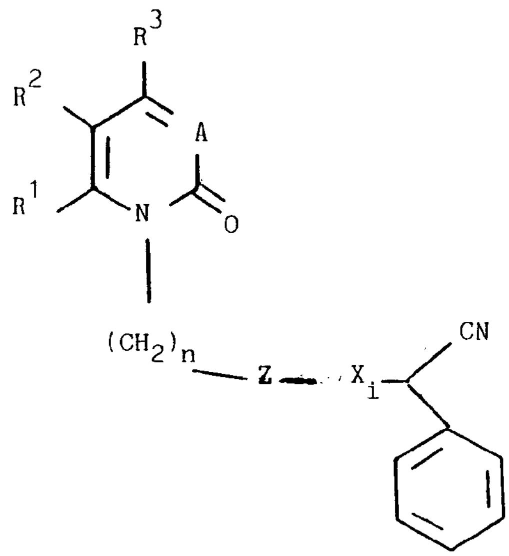

IX

in which A, Z, Y, $R^1$, $R^2$, $R^3$ and n are as defined above, and
$X_i$ is -O-, -S-, or

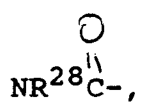

with an azide.

j) preparation of a compound of formula I, in which one of $R^4$ and $R^{20}$ is -COOH, which comprises hydrolysing a compound of formula VIII or IX,

k) preparation of a compound of formula I in which $R^5$ is $-NO_2$ which comprises nitrating a compound of formulae I, VIII or IX, in which $R^5$ is hydrogen, and where necessary hydrolysing or forming a tetrazole of the resulting compound,

l) preparation of a compound of formula I in which one or more hydroxy, amino or carboxylic acid groups is alkylated or acylated which comprises reacting an appropriate compound of formula I in which the hydroxy, amino or carboxylic acid group is unalkylated or unacylated with an alkylating or an acylating group,

m) production of a pharmaceutically acceptable salt, ester, amide, enantiomer or tautomer of a compound of formula I, by treating a compound of formula I, or another salt, an ester or an amide thereof, with a compound containing an available pharmaceutically acceptable ion and capable of converting the compound of formula I or the other salt, ester, amide, enantiomer or tautomer thereof, to a pharmaceutically acceptable salt of the compound of formula I.

**10.** A compound of formulae VIII or IX,

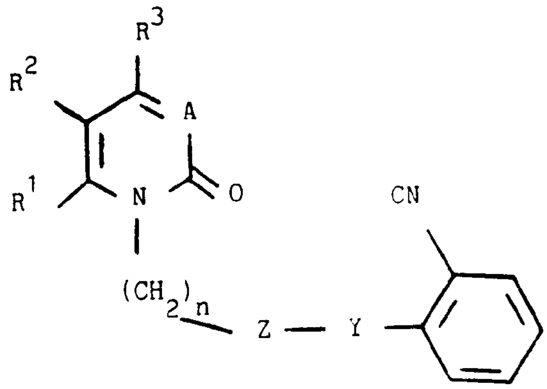

VIII

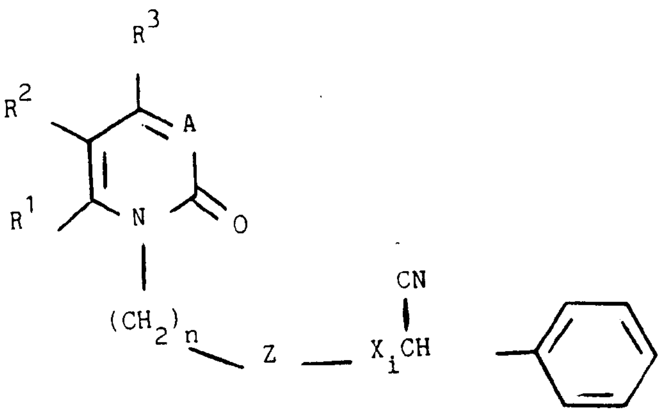

IX

in which A, Z, Y, $R^1$, $R^2$, $R^3$ and n are as defined in Claim 1, and $X_i$ is -O-, -S- or

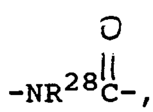

or a salt, ester, amide, tautomer or enantiomer thereof.

European Patent Office

# EUROPEAN SEARCH REPORT

Application Number

EP 92 30 1283

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A,D,<br>P | EP-A-0 434 249 (GLAXO GROUP LIMITED)<br>* claims 1,16,18,19 *<br>--- | 1-9 | C07D239/36<br>C07D213/64<br>C07D213/69<br>C07D213/80<br>C07D213/82<br>C07D215/22<br>C07D401/10<br>C07D401/12<br>C07D403/10<br>C07D405/06<br>C07D405/14<br>A61K31/505<br>A61K31/47<br>A61K31/455<br>A61K31/44 |
| A | EP-A-0 411 766 (MERCK & CO. INC.)<br>* claims 1,5; examples *<br>--- | 1,7,8 | |
| A | EP-A-0 407 342 (CIBA-GEIGY AG)<br>* claims 1,27,28; examples *<br>--- | 1,7,8 | |
| X | CHEMICAL ABSTRACTS, vol. 116, no. 17, 27 April 1992, Columbus, Ohio, US; abstract no. 174009P, K. KATANO ET AL.: 'Preparation of pyridine derivatives as angiotensin II antagonists.' page 848 ;<br>* CAS RN 139958-42-2*<br>* abstract * | 1,7,8 | |
| P,X | & WO-A-9 119 697 (MEIJI SEIKA KK) 26 December 1991<br>----- | | |
| | | | TECHNICAL FIELDS SEARCHED (Int. Cl.5)<br>C07D<br>A61K |

The present search report has been drawn up for all claims

| Place of search | Date of completion of the search | Examiner |
|---|---|---|
| THE HAGUE | 22 MAY 1992 | P. BOSMA |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)